Europäisches Patentamt

European Patent Office

Office européen des brevets

(19)

(11) **EP 0 338 189 B1**

(12) **EUROPÄISCHE PATENTSCHRIFT**

(45) Veröffentlichungstag und Bekanntmachung des Hinweises auf die Patenterteilung:
**24.04.1996 Patentblatt 1996/17**

(51) Int Cl.6: **G01N 33/92**
// C12Q1/60

(21) Anmeldenummer: **89100828.6**

(22) Anmeldetag: **18.01.1989**

(54) **Verfahren zur Herstellung affin-enzymatischer Verbindungen für den visuellen Nachweis von Cholesterin auf der Hautoberfläche eines Patienten auf der Basis eines Nachweismittels, das ein Cholesterinaffinans ist, und eines Visualisierungsmittels**

Method of producing affin-enzymatic compounds, for the visual detection of cholesterol on the surface of the skin of a patient, based on a detecting agent with an affinity for cholesterol and a visualisation agent

Méthode de production de combinaisons chimiques d'affin-enzymes pour la détection visuelle de cholestérol sur la surface de la peau d'un patient comprenant un agent chimique avec affinité pour cholestérol et un agent chimique de visualisation

(84) Benannte Vertragsstaaten:
**AT CH DE FR GB IT LI SE**

(30) Priorität: **19.01.1988 SU 4357046**

(43) Veröffentlichungstag der Anmeldung:
**25.10.1989 Patentblatt 1989/43**

(73) Patentinhaber: **2860601 Canada Inc.**
**Toronto, Ontario M4P 1E2 (CN)**

(72) Erfinder:
- **Lopukhin, Jury Mikhailovich, Dr.**
**Moscow (SU)**
- **Zuevsky, Viktor Viktorovich**
**Moscow (SU)**
- **Rabovsky, Alexandr Borisovich**
**Moscow (SU)**
- **Andrianova, Irina Pavlovna (180189)**
**deceased (SU)**

(74) Vertreter: **von Füner, Alexander, Dr. et al**
**Patentanwälte v. Füner, Ebbinghaus, Finck**
**Mariahilfplatz 2 & 3**
**D-81541 München (DE)**

(56) Entgegenhaltungen:
EP-A- 0 154 664          WO-A-84/00888
US-A- 4 071 020

- **JOURNAL OF ANTIBIOTICS, Band XXVII, Nr. 12, 1975, US; I.P. KATZENSTEIN et al., Seiten 943-951**
- **CHEMICAL ABSTRACTS, Band 96, 1982, Columbus, OH (US); A.N. KLIMOV et al., Seite 296, Nr. 176584q**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft das Gebiet der bioorganischen Chemie, und zwar ein Verfahren zur Herstellung affin-enzymatischer Verbindungen für den visuellen Nachweis von Cholesterin auf der Hautoberfläche eines Patienten auf der Basis eines Nachweismittels, das ein Cholesterinaffinans ist, und eines Visualisierungsmittels.

Am wirksamsten können die nach dem erfindungsgemäßen Verfahren hergestellten Verbindungen in der Diagnostik verwendet werden, darunter auch zur Früherkennung von Atherosklerose, zur Präzisierung der Diagnose unter klinischen, ambulanten und häuslichen Bedingungen sowie zur Kontrolle des Behandlungsverlaufs. Die angeführten Verbindungen können außerdem bei physiologischen, histologischen und histochemischen Untersuchungen zur Feststellung von erhöhtem Gehalt an Cholesterin sowie zu seiner Lokalisierung verwendet werden. Außerdem ermöglichen die Verbindungen auch die Feststellung von vermindertem Cholesteringehalt bei Patienten mit bestimmten Pathologien, insbesondere onkologischen Charakters.

Die Atherosklerose und ihre Komplikationen wie Infarkte, Insulte und Gangränen sind eine der Hauptursachen für die Sterblichkeit der Bevölkerung in allen Ländern der Welt.

Mehrjährige Untersuchungen haben ergeben, daß die Atherosklerose zu jenen Krankheiten zählt, die in erster Linie festgestellt und verhütet werden müssen, wobei der Hauptfaktor für das Atheroskleroserisiko die Cholesterinakkumulierung im Organismus ist. Die Atheroskleroseprophylaxe in der Bevölkerung setzt in erster Linie voraus, daß unter den Patienten die am meisten gefährdete Risikogruppe festgestellt wird, wonach diese differenziert zu behandeln ist und die betreffenden Patienten ihre Lebensweise ändern. Die Feststellung von Patientengruppen mit erhöhtem Atheroskleroserisiko aufgrund der im Organismus akkumulierten Cholesterinmenge ist das schwierigste am Problem der Atheroskleroseprophylaxe. Die bekannten Methoden der Atherosklerosediagnostik beruhen auf der quantitativen Bestimmung des Gesamtcholesterins im Plasma des venösen Blutes, (siehe Consensus Conference on Lowering Blood Cholesterol to Prevent Heart Desease, JAMA, 1985, 253, S. 2080 - 2086; The Lipid Research Clinics Population Studies Data Book; Publication 80 - 152, Bethesda, Ma., National Institutes of Health, 1980, Bd. 1; Lipid Research Clinics Programm, JAMA, 1984, 251, S. 351 - 364.) Ein Cholesteringehalt von über 260 mg gilt in bestimmten Fällen als ausreichend, um den Patienten zur Risikogruppe zu rechnen. Eine detailliertere Diagnose kann aufgrund der Analyse der Blutplasmalipoproteine und des Atherogenitätsindex gestellt werden, welcher das Verhältnis der Differenz von Gesamtcholesterin ($Ch_{ges}$) und Cholesterin der Lipoproteine von hoher Dichte ($Ch_{lhd}$) zum Cholesterin der Lipoproteine von hoher Dichte darstellt:

$$I_{ather.} = \frac{Ch_{ges} - Ch_{lhd}}{Ch_{lhd}}$$

Bei einem Atherogenitätsindex von über 3 wird der Patient zur Risikogruppe gerechnet und bei einem Index von über 5,6 zu den Atherosklerosepatienten (siehe Klimov A.N., "Fenotipirovanie lipoproteinov", Metodiceskie rekomendacii MZ SSSR, M., Medicina, 1975; Goldfourt V., Holtzman E., Neufeld H.N., Total and High Density Lipoprotein Cholesterol in the Serum and Risk of Mortality, British Medical Journal, 1985, 290, S. 1239 - 1243).

Die Anwendung dieser Methoden bedingt eine Blutentnahme, was für den Patienten traumatisch und außerdem nicht ungefährlich ist, wenn man dabei die Möglichkeit der Übertragung verschiedener Virusinfektionen berücksichtigt. Die Fraktionierung der Plasmalipoproteine und die Cholesterinanalyse stellen nach wie vor ein kompliziertes und teures Verfahren dar. Außerdem stehen in einem von drei Fällen das ermittelte Gesamtcholesterin und sogar die Ergebnisse einer vollständigen Phenotypierung nicht in Korrelation zur Schwere der Atherosklerose (siehe Mjasnikov A.L., "Gipertoniceskaja bolezn'", 1965, M., Medicina, S. 300).

Überdies haben die in den letzten Jahren durchgeführten Untersuchungen ergeben, daß das Blutplasma nicht in vollem Umfange die Prozesse der Cholesterinakkumulierung widerspiegeln kann, wie sie für die Arterienwandung und andere bradytrophe Gewebe kennzeichnend sind.

Eine weitere Verbesserung der Diagnoseverfahren ergab sich aus der Lösung einiger grundsätzlicher Fragen der Pathogenese der Atherosklerose. Es konnte gezeigt werden, daß das Gewebecholesterin die entscheidende Rolle bei der Entwicklung der Atherosklerose spielt. Es konnten Gewebe festgestellt werden, die analog der Arterienwandung das Cholesterin akkumulieren.

Untersuchungen der letzten Jahre haben ergeben, daß zwischen dem Cholesteringehalt in der Arterienwandung und in der Haut eine enge Korrelation besteht. Dies ermöglichte die Entwicklung grundsätzlich neuer Diagnoseverfahren, insbesondere für die Atherosklerose.

Die erwähnte Korrelation zwischen dem Cholesteringehalt in der Arterienwandung und in der Haut wurde durch unmittelbare Bestimmung des Cholesterins in Hautbioptaten ermittelt. Die Hautproben wurden in flüssigem Stickstoff eingefroren und lyophilisiert, wonach man das Cholesterin mit Folch-Reagens extrahierte und mit konventionellen chemischen bzw. biochemischen Methoden bestimmte (siehe Nikitin Ju.P., Gordienko I.A., Dolgov A.V., Filimonova T. I., "Soderzanie cholesterina v koze i ego korreljacija s lipidnym pokazatelem syvorotki krovi u zdorovych ljudej i bol'nych isemiceskoj bolezn'ju serdca", Kardiologija, 1987, XXVII, Nr. 10, S. 48 - 51; Bouisson H., De Graeve, Solera M.L. et al. "Ann. Biol. Clin." 1982, Bd. 40, S. 361 - 407).

Aufgrund ihres traumatischen Charakters und der Kompliziertheit ihrer Durchführung ist diese Methode allerdings für Reihenuntersuchungen ungeeignet.

Die US-PS 4458686 beschreibt ein Verfahren zur Bestimmung von im Blut lokalisierter Glukose bzw. Ethanol unmittelbar unter der Haut und auf der Hautoberfläche. Angegeben wird auch die Möglichkeit der Bestimmung von Cholesterin unter Verwendung von Cholesterinoxidase. Dem Verfahren zur Bestimmung der Menge dieser Stoffe liegt die stöchiometrische Änderung der Sauerstoffkonzentration bei Einsatz für das festzustellende Substrat spezifischer Redoxfermente, vorzugsweise Oxidoreduktase zugrunde. Dabei erfolgt die quantitative Messung der Änderung der Sauerstoffkonzentration elektrochemisch wie zum Beispiel polarographisch mit speziellen Geräten und einer eigens dafür entwickelten Elektrode. Die dafür erforderlichen komplizierten Geräte bedürfen zur Durchführung der Diagnose eines hochqualifizierten Personals. All dies führt zwangsläufig zu einer Einschränkung der Anwendbarkeit dieses Verfahrens bei Reihenuntersuchungen.

Aus der publizierten PCT/US-Anmeldung 84/00888 ist ein Komplex aus einem Nachweis- und Visualisierungsmittel bekannt, bei dem diese unmittelbar oder über ein Vernetzungsmittel miteinander verbunden sind. Dieser Komplex dient zur Bestimmung geringer Mengen spezifischer Moleküle, wie zum Beispiel von Lipiden in biologischen Geweben. Dieses Verfahren zur Ermittlung von Lipiden läßt sich jedoch nur unter Laborbedingungen anwenden und erfordert, daß dem Patienten vorgängig eine biologische Flüssigkeit oder ein Gewebe entnommen wird, was bedeutet, daß das Verfahren an sich traumatisch, mehrstufig und kompliziert in seiner Durchführung ist.

Daten zur Korrelation zwischen dem Cholesteringehalt in der Haut und dem Grad der atherosklerotischen Schädigung der Gefäße erhält man an Hautbioptaten. Dieses Verfahren ist nicht nur traumatisch, sondern hat auch noch eine Reihe weiterer Nachteile, da 1 mm dicke Bioptate verschiedene Hautschichten umfassen, und zwar die Hornschicht (durchschnittliche Dicke 0,1 mm) und Bindegewebe, das heißt die eigentliche Haut (Derma), die wiederum zwei Schichten umfaßt, und zwar das Stratum papillare und das Stratum reticulare. Diese beiden Schichten werden gut mit Blut versorgt, weshalb entsprechende Bioptate auch Gefäße und Blut enthalten und außerdem noch Schweiß- und Talgdrüsen sowie ihre Sekrete. Unmittelbar unter dem Derma befindet sich das Unterhautfettgewebe, das ebenfalls zum Bioptat gehören kann. Die Inhomogenität der Bioptate kann somit die Ergebnisse der Bestimmung des in der Haut akkumulierten Cholesterins verzerren. In dieser Hinsicht ist ein Verfahren am genauesten, welches die Ermittlung des Cholesteringehalts auf der Hautoberfläche in der Hornschicht des Dermas ermöglicht.

Der Erfindung liegt die Aufgabe zugrunde, ein Verfahren zur Herstellung von affin-enzymatischen Verbindungen für den visuellen Nachweis von Cholesterin unmittelbar auf der Haut des Patienten, und zwar insbesondere in der Hornschicht der Epidermis zu entwickeln, ohne dem Patienten Blut- und Hautbioptate entnehmen zu müssen. Für die Diagnose kann jeder beliebige Hautbezirk verwendet werden. Am geeignetsten ist jedoch die Handfläche, da diese keine Talgdrüsen enthält, deren Ausscheidungen ebenso wie die Hornschicht der Haut Cholesterin enthalten, das die Ergebnisse der Diagnose beeinflussen kann.

Diese Aufgabe wurde erfindundungsgemäß durch Verfahren zur Herstellung von affin-enzymatischen Verbindungen gelöst, die bifunktionelle Verbindungen darstellen. Diese verbinden sich selektiv mit dem freien Cholesterin der Haut des Patienten und können dann visuell sichtbar gemacht werden. Für die Herstellung einer solchen Verbindung sind wenigstens zwei Komponenten erforderlich, und zwar ein Nachweismittel A, ausgewählt aus der Gruppe von Stoffen, die mit dem freien Cholesterin der Haut zur Adressierung der gesamten bifunktionellen Verbindung auf das freie Cholesterin der Haut mit diesem selektiv einen festen Komplex zu bilden vermögen, und ein Visualisierungsmittel B, das die mit dem Cholesterin verknüpfte bifunktionelle Verbindung visuell sichtbar zu machen vermag.

Die erfindungsgemäß herstellbaren Verbindungen führen zu einer maximalen Vereinfachung der Atherosklerosediagnostik, sind nicht traumatisch und erfordern auch keinen speziellen apparativen Aufwand. Die Anwendung dieser Verbindungen zur Diagnose der Atherosklerose ermöglicht es, die untersuchten Personen einer der drei folgenden Gruppen zuzuordnen: an Atherosklerose erkrankte Personen, Risikogruppe und Gesunde.

Die erfindungsgemäß herstellbaren affin-enzymatischen Verbindungen für den visuellen Nachweis von Cholesterin und das auf ihrer Verwendung beruhende Diagnoseverfahren ("Dreipunktverfahren") sind auch für Reihenuntersuchungen geeignet Das Verfahren ist sehr einfach anzuwenden und bedarf keiner speziellen Qualifikation seitens des medizinischen Personals; es kann sogar zu Hause durchgeführt werden.

Die nach den erfindungsgemäßen Verfahren hergestellten Verbindungen können auch aus dem Nachweismittel A und dem Visualisierungsmittel B durch chemische Aktivierung der funktionellen Gruppen eines der beiden Mittel hergestellt werden. In diesem Falle kommt es, nachdem man einem der beiden Mittel, das die aktivierten chemischen Gruppen enthält, das jeweils andere Mittel zugesetzt hat, bei bestimmten Mengenverhältnissen zwischen den Mitteln A und B zur Bildung einer festen chemischen Bindung, wodurch man höhermolekulare bifunktionelle Verbindungen für den visuellen Nachweis des Cholesterins auf der Haut des Patienten erhält.

Als Nachweismittel A, das ein Cholesterinaffinans darstellt, kommen folgende Verbindungen in Frage:

Steroidglykoside, die als Aglykon das Cyclopentanperhydrophenantrenfragment der Furostanol- und Spirostanolreihe sowie ein Oligosaccharidfragment mit 3 bis 10 unverzweigten oder verzweigten Monosacchariden enthalten (siehe P.K. Kintja, "Stroenie i biologiceskaja aktivnost' steroidnych glikozidov rjada spirostana i furostana", Kisinev, Vlg. Stinca, 1987, S. 142) oder

Triterpenglykoside, die ein Aglykon der α- oder β-Amyryl-, Lupan-, Hopan-, Dammaran-, Linostan- oder Holostanreihe und ein Oligosaccharid aus 2 bis 8 verzweigten oder unverzweigten Resten enthalten (s. G.E. Dekanosidze, V.Ja. Cirva, T.V. Sergienko, N.I. Uvarova, "Issledovanie triterpenovych glikozidov", Tbilisi, Vlg. Mecniereba, 1982) oder

Hydrophobe Proteine, die mit dem Cholesterin selektiv eine Komplexverbindung zu bilden vermögen (s. 1. A.N. Klimov, G.V. Titova, K.A. Kozevnikova, Biochimija, 1982, Bd. 47, Nr. 2, S. 226 - 232, 2. A.N. Klimov, K.A. Kozevnikova, N.N. Kljueva et al. Voprosy med. chimii, 1984, Bd. 30, Nr. 3, S. 86 - 90; 3. G.V. Titova, N.N. Kljueva, K.A. Kozevnikova et al., Biochimija, 1980, Bd. 45, Nr. 1, S. 51 - 55) oder

eiweißartige Toxine, die aus Bakterien, marinen Mikroorganismen, Insketen oder Schlangen gewonnen werden und mit dem Cholesterin selektiv eine Komplexverbindung zu bilden vermögen (s, M.V. Dalin, N.G. Fis, "Belkovye toksiny mikrobov", Moskau, Vlg. "Medicina", 1980) oder

Polyenantibiotika, die mit dem Cholesterin selektiv eine Komplexverbindung zu bilden vermögen (s, 1. J.P. Katzenstein, A.M. Spielvogel, A.W. Norman "J. Antibiot." 27, 12, 1974, S. 943 - 951; 2. Jong Shang-Shyng, Wang Hsi--Hua "Clin. J. Microbiol.", 1976, 9, /1 - 2/, S. 19 - 30; 3. Readig Josephine D. et al., "Biochim. Biophis. Acta", 1982, 685 /2/, S. 219 - 224) oder

hochaffine Enzyme, die Cholesterin als Substrat aufweisen und zu diesem eine hohe Affinität zeigen.

Als Visualisierungsmittel B kommen folgende Enzyme in Frage: Acetylcholinesterase, Tyrosinase, Glukoso-6-phosphatdehydrogenase, Glukooxidase, Glukoamylase, Galaktosidase, Peroxidase, alkalische oder saure Phosphatase, α-Chymotrypsin oder Pyrophosphatase.

Gegenstand der Erfindung ist unter anderem ein Verfahren zur Herstellung von affin-enzymatischen Verbindungen zum visuellen Nachweis auf der Hautoberfläche eines Patienten auf der Basis eines Nachweismittels A, das ein Affinans für Cholesterin darstellt, und eines Visualisierungsmittels B, dadurch gekennzeichnet, daß das Nachweismittel A, das ausgewählt ist aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder aus der Gruppe der Triterpenglykoside, die ein Aglykon der α-oder β-Amyryl-, Lupan-, Hopan-, Dammaran-, Linostan- oder Holostanreihe und ein Oligosaccharid aus 2 bis 8 verzweigten oder unverzweigten Resten enthalten, oder
aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen oder aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder
aus der Gruppe der Polyenantibiotika Amphotericin B, Philipin oder Nistatin, oder
aus der Gruppe der Enzyme, die aus Cholesterinoxidasen, Cholesterinhydrogenasen und Cholesterinesterasen besteht,
durch chemische Methoden in wäßrigem Medium bei einem Molarverhältnis Nachweismittel A : Aktivator - 1:1 bis 1: 10 und einer Konzentration des Nachweismittels A von 1 bis 20 mg/ml bei einer Temperatur von 0 bis 25°C und einem pH-Wert von 4 bis 11 1 bis 24 Stunden lang chemisch aktiviert wird und die erhaltene Lösung mit dem Visualisierungsmittel B, ausgewählt aus der Gruppe der Enzyme Acetylcholinesterase, Tyrosinase, Glukoso-6-phosphatdehydrogenase, Glukosooxidase, Glukosoamylase, Galaktosidase, Peroxidase, alkalische oder saure Phosphatase, α-Chymotrypsin oder Pyrophosphatase, im Molarverhältnis A:B = 20:1 bis 1:1 versetzt und die erhaltene Lösung 1 bis 24 Stunden lang bei einer Temperatur von 0 bis 25°C gehalten wird.
Wie bereits oben ausgeführt wurde, können auch die funktionellen Gruppen des Visualisierungsmittels B vorgängig chemisch aktiviert werden. In diesem Falle erfolgt die chemische Aktivierung des Visualisierungsmittels B im wäßrigen Medium bei einem Molarverhältniz von Visualisierungsmittel B zu Aktivierungsmittel von 1:1 bis 1:10, einer Visualisierungsmittelkonzentration von 1 bis 20 mg/ml, einer Temperatur von 0 bis 25°C und einem pH-Wert von 4 bis 11 im Laufe von 1 bis 24 Stunden, wonach der erhaltenen Lösung das Nachweismittel A, das eine der oben genannten Substanzen darstellt, bei einem Molarverhältnis von A:B von 20:1 bis 1:1 zugesetzt wird und die Lösung bei einer Temperatur von 0 bis 25°C 1 bis 24 Stunden lang bis zum Erhalt des Endprodukts gehalten wird.

Die chemische Aktivierung des Nachweismittels A bzw. des Visualisierungsmittels B erfolgt nach der an sich bekannten Azid-, Carbodiimid- oder Succinimidmethode bzw. nach der Methode der Perjodatoxidation.

In den Fällen, in denen als Visualisierungsmittel B eine höhermolekulare Verbindung und zwar ein Enzym verwendet wird und als Nachweismittel A niedermolekulare Verbindungen wie zum Beispiel Glykoside verwendet werden, kann das Molekül des Endproduktes nicht mehr als 1 Molekül Visualisierungsmittel B aufweisen. Dabei darf der Molargehalt an Nachweismittel A, das die Bindung an das Cholesterin der Haut im Endprodukt ermöglicht, den Gehalt an Visualisierungsmittel nur um einige Male übersteigen, und zwar entsprechend dem Gehalt an funktionellen Gruppen im Enzymmolekül, die für die Verknüpfung mit dem Nachweismittel A ohne erheblichen Verlust an enzymatischer Aktivität geeignet sind. Bei solchen Verbindungen ist der Gehalt an Enzym, das als Visualisierungsmittel B verwendet wird, beschränkt. Daraus folgt die relativ geringe Empfindlichkeit der erhaltenen Verbindungen, weshalb bei der Diagnose die Haut länger exponiert werden muß.

Für die Erweiterung der technischen Möglichkeiten des Einsatzes affin-enzymatischer Verbindungen für den visuellen Nachweis auf der Haut des Patienten und zur Steigerung ihrer Empfindlichkeit wird gemäß den Ansprüchen 3 und 4 vorgeschlagen, das Nachweismittel A mit dem Visualisierungsmittel B mit Hilfe eines Vernetzungsmittels C, ausgewählt unter niedermolekularen asymmetrischen bifunktionellen Verbindungen wie Bromcyan, Trichlortriazin oder 2-Amino-4,6-dichlor-S-triazin zu verknüpfen. Dabei können die Verbindungen auf zweierlei Weise hergestellt werden, und zwar kann entweder zuerst das Nachweismittel A mit dem Vernetzungsmittel C verbunden werden, wonach zum Erhalt des Endprodukts dem intermediären Produkt (A + C) das Visualisierungsmittel B zugesetzt wird oder es wird zuerst das intermediäre Produkt (B + C) gebildet, dem dann das Nachweismittel A zugesetzt wird. Im ersteren Falle erhält man die affin-enzymatischen Verbindungen durch gewöhnliches Lösen in einer wäßrigen Salz-Pufferlösung (pH 5 bis 9) des Nachweismittels A, ausgewählt unter den angeführten Substanzen, bei einer Konzentration von 1 bis 20 mg/ml, wonach man der Lösung das niedermolekulare asymmetrische bifunktionelle Vernetzungsmittel C, ausgewählt aus der Gruppe Bromcyan, Trichlortriazin oder 2-Amino-4,6-dichlor-S-triazin bei einem Molarverhältnis von A:C von 1:0,5 bis 1:10 zusetzt, die erhaltene Lösung bei 0 bis 20°C 1 bis 20 Stunden lang hält, danach das Visualisierungsmittel B, ausgewählt aus den oben genannten Substanzen, bei einem Molarverhältnis von A:B von 20:1 bis 1:1 zusetzt und die Lösung bei 0 bis 20°C 1 bis 48 Stunden lang bis zum Erhalt des Endprodukts hält.

Bei der Herstellung der affin-enzymatischen Verbindungen über das intermediäre Produkt B + C löst man das aus den oben genannten Substanzen gewählte Visualisierungsmittel B auf herkömmliche Weise in einer wäßrigen Salz-Pufferlösung (pH 5 bis 9) bei einer Visualisierungmmittelkonzentration von 1 bis 20 mg/ml, wonach man der Lösung das niedermolekulare asymmetrische bifunktionelle Vernetzungsmittel C, ausgewählt unter Bromcyan, Trichlortriazin oder 2-Amino-4,6-dichlor-S-triazin, bei einem Molarverhältnis von B:C von 1:0,5 bis 1:10 zusetzt, die erhaltene Lösung bei 0 bis 20°C 1 bis 20 Stunden hält, danach das Nachweismittel A, ausgewählt aus den obengenannten Substanzen, bei einem Molarverhältnis von A:B von 20:1 bis 1:1 zusetzt und die Lösung bei 0 bis 20°C 1 bis 48 Stunden lang bis zum Erhalt des Endprodukts hält.

Die maximale Empfindlichkeit der affin-enzymatischen Verbindungen für den visuellen Nachweis des Cholesterins auf der Haut des Patienten erzielt man allerdings nur dann, wenn man als Vernetzungsmittel höhermolekulare polyfunktionelle Verbindungen wie Polysaccharide, Proteine oder synthetische Polymere verwendet, das heißt beliebige höhermolekulare Verbindungen, die eine beliebige funktionelle Gruppe enthalten, ausgewählt aus der Reihe primäres Amin, Carboxyl, Hydroxyl, Aldehyd, Hydrohalogenid, Mischanhydrid, Iminoether, Azid, Hydrazid, Maleimid, Isocyanat oder Epoxid. Dabei werden auf einer dieser Verbindungen unabhängig voneinander das Nachweismittel A, das eines der angeführten Stoffe oder deren Gemische darstellt, und das Visualisierungsmittel B immobilisiert, das ebenfalls eines der angeführten Stoffe darstellt. Eine derartige affin-enzymatische Verbindung für den visuellen Nachweis von Cholesterin auf der Hautoberfläche des Patienten erlaubt es, das Verhältnis von Nachweismittel A zu Visualisierungsmittel B je nach dem Grad der Affinität des gewählten Nachweismittels A gegenüber dem Cholesterin der Haut bzw. der Aktivität des eingesetzten Enzyms B innerhalb eines sehr weiten Bereichs zu variieren. So kann zum Beispiel bei der Verwendung eines Nachweismittels A mit einer schwächeren Affinität gegenüber dem Cholesterin der Haut sein relativer Molargehalt im Endprodukt wesentlich erhöht werden.

Solche affin-enzymatischen Verbindungen werden dadurch hergestellt, daß man das Nachweismittel A, das einen der oben aufgezählten Stoffe darstellt oder Gemische davon, und das Visualisierungsmittel B, welches ebenfalls einen der oben aufgezählten Stoffe darstellt, in einem Molarverhältnis A:B von 20:1 bis 1:1 vorlegt, sie in einer wäßrigen Salz-Pufferlösung mit einem pH-Wert von 5 bis 9 bei einer Konzentration von A wie auch von B 0,1 bis 20 mg/ml löst, die erhaltene Lösung mit einem höhermolekularen polyfunktionellen Vernetzungsmittel D, das Polysaccharide, Proteine oder synthetische Polymere, d. h. beliebige höhermolekulare Verbindungen, die eine beliebige funktionelle Gruppe, ausgewählt aus der Reihe primäres Amin, Carboxyl, Hydroxyl, Aldehyd, Hydrohalogenid, Mischanhydrid, Azid, Hydrazid, Maleimid, Isocyanat oder Epoxid darstellt, in einem Molarverhältnis von A+B:D von 1:0,5 bis 1:10 versetzt und die erhaltene Lösung bei einer Temperatur von 0 bis 20°C 1 bis 20 Stunden lang bis zum Erhalt des Endprodukts hält.

Bevorzugte Steroidglykoside, die als Nachweismittel A verwendet werden und als Aglykon ein Cyclopentanperhydrophenantrenfragment der Furostanol- oder Spirostanolreihe sowie ein Oligosaccharidfragment mit 3 bis 10 unverzweigten oder verzweigten Monosacchariden enthalten, sind Agavosid A, Agavosid B, Agavosid C, Agavosid D, Agavosid F, Agavosid H, Agavosid G, Trillin, Funcosid C, Funcosid D, Funcosid F, Funcosid G, Funcosid I, Dioscin, Gracillin, Protodioscin, Cicubasaponin, Juccosid E, Juccosid H, Lanatigonin, Desglukodigitonin, Digitonin, Gitonin, Rocksosid C, Rocksosid D, Rocksosid E, Funcosid E, Alliumosid C, Polygonatosid, Tigogenintetraosid, Tigogeninhexaosid, Capsicosid, Ammumosid B, Ammumosid C, Ammumosid D, Ammumosid E, Desglukodesramnoparillin, Desglukoparillin, Parillin, Sarsaparillosid, Asparagosid C, Asparagosid D, Asparagosid G, Asparagosid H, Protoyuccosid H, Yukkosid B, Lanatigosid, , Purpureagitosid, Gekogenin, Rockogenin, Diosgenin, Tigogenin, Protopolygonatosid, Tomatonin, Karataviosid A, Acantofilosid C, Alliofurosid A, Alliospirosid A, Theasaponin, Acantofilosid B, Tigonin (gesamt) oder Glykosid der Pflanze Calha polypetala.

Unter den genannten Steroidglykosiden sind die Funcoside C, D, E, F, G oder I, Dioscin, Rockosid, Lanatigonin, Digitonin und Tomatonin besonders bevorzugt. Am meisten bevorzugt sind Digitonin und Tomatonin.

Bevorzugte Triterpenglykoside, die als Nachweismittel A verwendet werden und ein Aglykon der $\alpha$- oder $\beta$-Amyryl-, Lupan-, Hopan-, Dammaran-, Lanostan- oder Holostanreihe sowie ein Oligosaccharid aus 2 bis 8 verzweigten oder unverzweigten Resten enthalten, sind Aescin, Avenacin, Theasaponin, $\alpha$-Hederin, Caulosid C, Cyclamin, Chinovin, Saponine der Zuckerrübe, Gypsosid sowie Triterpenglykoside, die aus Pflanze Fatsia japonica gewonnen werden.

Hydrophobe Proteine, die als Nachweismittel A verwendet werden und die mit dem Cholesterin eine Komplexverbindung selektiv zu bilden vermögen, sind das Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen.

Eiweißartige Toxine, die als Nachweismittel A verwendet werden, mit dem Cholesterin selektiv eine Komplexverbindung zu bilden vermögen und aus Bakterien, marinen Mikroorganismen, Insekten oder Schlangen gewonnen werden, sind Streptolysin o, Pneumolysin, Listeriolysin, $\theta$-Toxin C.l., gewonnen aus Perfrigens Typus A und C, $\delta$-Toxin C.l. novyj Typus A und C, Hämolysin C.l. histolyticum, Hämolysin C.l. botulinum Typus C und D, Tetanolysin, Alveolysin, Türingeolysin oder Cytotoxin aus der Aktinie Metridium senile.

Polyenantibiotika, die als Nachweismittel A verwendet werden und mit Cholesterin eine Komplexverbindung zu bilden vermögen, sind Amphotericin B, Philipin oder Nistatin.

Hochaffine Enzyme, die eine hohe Affinität zu Cholesterin besitzen und als Nachweismittel A verwendet werden, sind Cholesterinoxidasen, Cholesterindehydrogenasen oder Cholesterinesterasen.

Unter den aufgeführten cholesterinaffinen Nachweismitteln A wie Glykosiden, hydrophoben Proteinen, eiweißartigen Toxinen, Polyenantibiotika und hochaffinen Enzymen sind die Glykoside bevorzugt, da sie chemisch am beständigsten sind und sowohl organischen Lösungsmitteln als auch hohen Temperaturen bei der Synthese standhalten, ohne daß sie ihre Fähigkeit, mit dem Cholesterin der Haut einen Komplex zu bilden, einbüßen. Das niedrige Molekulargewicht der Glykoside ermöglicht die Herstellung eines Mittels mit hohem Molargehalt an Nachweismittel, was eine Vielzahl von Punkten für die Wechselwirkung zwischen dem Mittel und dem Cholesterin der Haut gewährleistet.

Besonders bevorzugt unter den Glykosiden sind die Steroidglykoside, die im Vergleich zu den Triterpenglykosiden mit dem Cholesterin am wirksamsten einen Komplex zu bilden vermögen.

Die hydrophoben Proteine, eiweißartigen Toxine, Polyenantibiotika und hochaffinen Enzyme können außerdem mit Erfolg für die Herstellung eines diagnostischen Mittels verwendet werden, bewirken jedoch aufgrund ihrer geringen chemischen Beständigkeit und ihres Inaktivierungsvermögens gewisse Einschränkungen beim Verfahren zur Herstellung der affinenzymatischen Verbindungen. Außerdem vermindert das hohe Molekulargewicht dieser Nachweismittel etwas die Empfindlichkeit der Endverbindung, was für den Cholesterinnachweis auf der Haut eine erhöhte Konzentration des Mittels und längere Einwirkdauer erforderlich macht.

Man ist verständlicherweise bestrebt, die Reaktionen unter milden Bedingungen durchzuführen, das heißt bei neutralem pH, geringer Ionenstärke, niedrigen Temperaturen und bei verkürzter Reaktionsdauer.

Als Visualisierungsmittel B werden Enzyme verwendet, die aufgrund der Reaktion mit dem Nachweismittel A das affinenzymatische Produkt für den visuellen Nachweis von Cholesterin auf der Haut des Patienten liefern. Sie werden ausgewählt aus der Gruppe Acetylcholinesterase, Tyrosinase, Glukose-6-phosphatdehydrogenase, Glukoseoxidase, Glukosaamylase, Galaktosidase, Peroxidase, alkalische oder saure Phosphatase, $\alpha$-Chymotrypsin oder Pyrophosphatase.

Alle diese Enzyme können zusammen mit einem beliebigen oben aufgeführten Nachweismittel A verwendet werden, bevorzugt sind jedoch Steroidglykoside.

Die Verwendung eines Vernetzungsmittels (C) erweitert die technologischen Möglichkeiten des Verfahrens zur Herstellung einer Verbindung zum visuellen Nachweis von Cholesterin auf der Hautoberfläche eines Patienten, wobei die funktionelle Aktivität der Mittel A und B maximal erhalten bleibt.

Besonders bevorzugt sind Verbindungen, fur deren Herstellung als cholesterinaffines Nachweismittel A Steroidglykoside verwendet werden, die als Aglykon das Cyclopentanperhydrophenantrenfragment der Furostanol- und Spirostanolreihe sowie ein Oligosaccharidfragment mit 3 bis 10 unverzweigten oder verzweigten Monosacchariden enthalten.

Derartige Steroidglykoside besitzen hohe chemische Beständigkeit und können unter scharfen Bedingungen, das heißt bei hohen Temperaturen und gegebenenfalls durch Lösen in organischen Lösungsmitteln auf einem Vernetzungspolymer immobilisiert werden, was ihren hohen Gehalt im Endprodukt gewährleistet. Die Verbindung für den visuellen Nachweis von Cholesterin auf der Hautoberfläche des Patienten erhält man durch Immobilisierung des Nachweismittels A auf dem Vernetzungsmittel C unter scharfen Bedingungen und nachfolgende Immobilisierung des Visualisierungsenzyms B auf dem das Vernetzungsmittel C und das Nachweismittel A enthaltenden Produkt unter schonenden Bedingungen, damit seine enzymatische Aktivität erhalten bleibt.

Als Vernetzungsmittel C können asymmetrische niedermolekulare bifunktionelle Verbindungen, wie zum Beispiel Bromcyan, Trichlortriazin oder 2-Amino-4,6-dichlor-S-triazin verwendet werden.

Die Verwendung höhermolekularer polyfunktioneller Verbindungen als Vernetzungsmittel D ermöglicht es, das Verhältnis von Nachweismittel A zu Visualisierungsmittel B in der Verbindung zum visuellen Nachweis von Cholesterin innerhalb eines weiten Bereichs zu variieren, um zum Beispiel zu einem optimalen Verhältnis zwischen der Zahl der Verknüpfungszentren der Verbindung mit der Hautoberfläche (Nachweismittel A) und der Menge an Visualisierungsmittel B zu gelangen.

Als höhermolekulares polyfunktionelles Vernetzungsmittel D verwendet man Polysaccharide, Proteine oder synthetische Polymere, das heißt beliebige höhermolekulare Verbindungen, die eine beliebige funktionelle Gruppe, ausgewählt aus der Reihe primäres Amin, Carboxyl, Hydroxyl, Aldehyd, Halogenanhydrid, Mischanhydrid, Iminoether, Azid, Hydrazid, Maleimid, Isocyanat oder Epoxid enthalten.

Als höhermolekulares polyfunktionelles Vernetzungsmittel D kommen bevorzugt Acrylsäure- oder Maleinsäureanhydrid-Copolymerisate in Frage.

Besonders bevorzugt als höhermolekulares polyfunktionelles Mittel D ist das N-Vinylpyrrolidon-Maleinsäureanhydrid-Copolymerisat. Die angeführten Vernetzungsmittel D werden mit einem beliebigen Nachweismittel A und einem beliebigen Visualisierungsmittel B verwendet. Dabei erhält man immer ein Endprodukt A-D-B.

Besonders bevorzugt sind Verbindungen, bei denen als Nachweismittel A Steroidglykoside verwendet werden.

Was das Mittel B betrifft, so werden hier die alkalische Phosphatase und Peroxidase bevorzugt. Besonders bevorzugt ist die Peroxidase des Krens (Meerrettichs).

Als Aktivierungsmittel für das Nachweismittel A und das Visualisierungsmittel B verwendet man Verbindungen, welche die funktionellen Gruppen dieser Mittel in ihre reaktionsfähige Form überführen. Gruppen wie primäres Amin, Carboxyl und $\alpha$-Glykol können durch an sich bekannte Methoden, wie die Azid-, Carbodiimid-, Succinimid- oder Perjodatmethode aktiviert werden.

Besonders bevorzugt sind die Perjodat- und die Carbodiimidmethode, da diese Methoden die maximal schonenden Synthesebedingungen gewährleisten und dabei eine maximale Erhaltung der Aktivität des immobilisierten Enzyms ermöglichen.

Als wäßrige Salzpufferlösungen verwendet man Lösungen, die aus Verbindungen erhalten wurden, welche eine Pufferwirkung in einem pH-Bereich von 5 bis 9 gewährleisten, und die keine Inaktivierung oder Inhibierung der Enzyme verursachen. Solche Lösungen können zum Beispiel Borat-, Zitrat-, Phosphat- und andere Pufferlösungen sein. Gewöhnlich verwendet man jedoch Lösungen mit einer Salzkonzentration, die während der Immobilisierung einen konstanten pH-Wert der Lösung gewährleistet, jedoch nicht Werte übersteigt, bei denen es zur Denaturierung des Enzyms kommt.

Einige der Nachweismittel A, wie Steroidglykoside, Triterpenglykoside und Polyenantibiotika lösen sich in Wasser nur schlecht oder überhaupt nicht, weshalb für ihre Lösung organische Lösungsmittel verwendet werden. Als solche kommen polare aprotische Lösungsmittel, wie zum Beispiel Dimethylsulfoxid, Dimethylformamid, Hexamethanol, ein Gemisch von Dimethylformamid mit Toluol (2:1) oder ein Gemisch von Dimethylformamid mit Hexan (2:1) in Frage.

Die Bedingungen für das Verfahren zur Herstellung der Verbindungen zum Cholesterinnachweis (Temperatur und Haltezeit in Thermostaten) hängen von der Wahl der Komponenten A, B und D ab. Werden als Visualisierungsmittel B Enzyme verwendet, darf die Reaktionstemperatur 20°C nicht übersteigen. Vorzugsweise wird die Reaktion bei 4°C in Pufferlösungen mit einem pH-Wert von 5 bis 9 durchgeführt. Werden synthetische Polymere als Vernetzungsmittel D und Glykoside als Nachweismittel A verwendet, kann die Reaktion in organischen Lösungsmitteln und bei hohen Temperaturen (bis 120°C) durchgeführt und eine Reaktionsdauer gewählt werden, die für die Erzielung einer maximalen Ausbeute an Endprodukt ausreicht.

Die Molarverhältnisse der Reaktionskomponenten werden entsprechend dem Molekulargewicht der gewählten Mittel A bzw. B, dem Grad der Affinität gegenüber Cholesterin der Haut und der Aktivität des gewählten Enzyms gewählt.

Nachfolgend wird die Erfindung anhand von Ausführungsbeispielen und Zeichnungen, auf denen sie dargestellt ist, näher erläutert. Dabei zeigen:

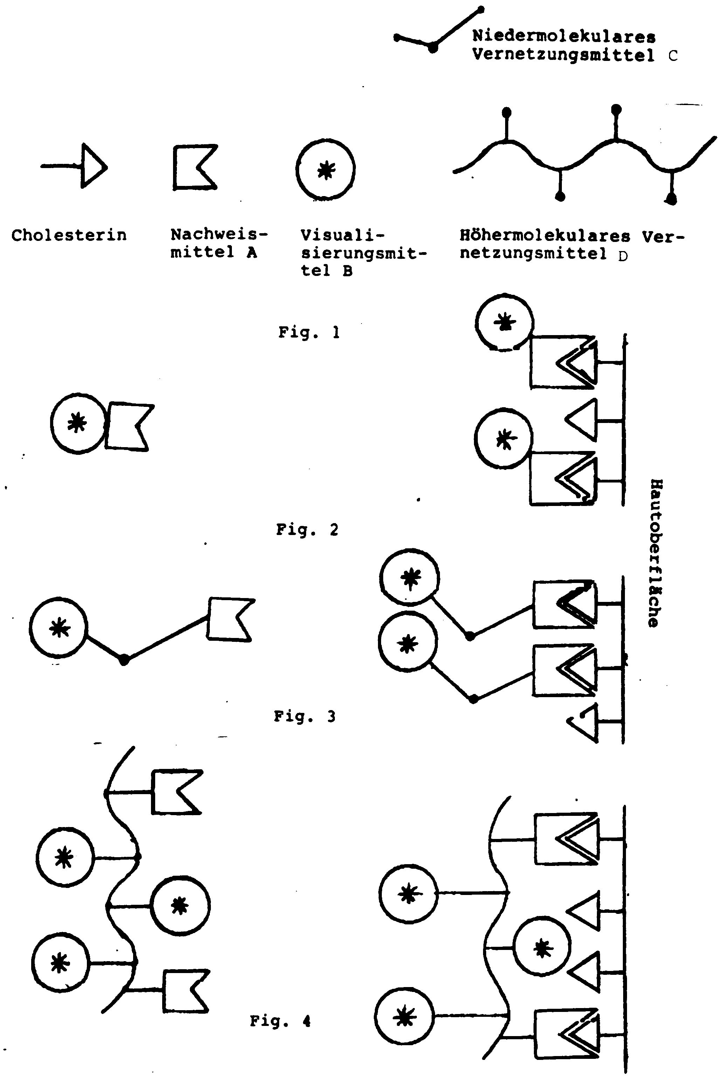

Fig. 1    die Bezugszeichen,

Fig. 2    die erhaltenen Verbindungen und ihre Wechselwirkung mit dem Cholesterin auf der Hautoberfläche des Patienten gemäß den Ansprüchen 1 und 2,

Fig. 3 die erhaltenen Verbindungen und ihre Wechselwirkung mit dem Cholesterin auf der Hautoberfläche des Patienten gemäß den Ansprüchen 3 und 4 und

Fig. 4 die erhaltenen Verbindungen und ihre Wechselwirkung mit dem Cholesterin auf der Hautoberfläche des Patienten gemäß den Ansprüchen 5 und 16.

Beispiel 1

100 mg Tomatonin (A) werden in 10 ml Wasser vorgelegt, wonach man 40 mg Natriumper jodat zusetzt und bei 20°C 4 Stunden lang hält. Danach wird 1 ml der erhaltenen Lösung mit 1 ml einer Lösung von $\alpha$-Chymotrypsin (B) (10 mg/ml) in einem 0,2 M Phosphatpuffer mit einem pH-Wert von 7,5 versetzt und das Gemisch 12 Stunden bei 4°C gehalten, wodurch man eine wäßrige Lösung des Endprodukts erhält.

Beispiel 2

3 mg Peroxidase (B) des Krens werden in 1 ml Wasser gelöst und mit 0,5 mg Natriumperjodat versetzt, wonach man das Gemisch 8 Stunden bei 4°C hält. Die erhaltene Lösung wird mit 3 mg Cholesterinoxidase (A) und 1 ml eines 0,2 M Phosphatpuffers mit einem pH-Wert von 7,5 versetzt und 12 Stunden bei 4°C gehalten, wodurch man eine Lösung des Endprodukts erhält.

Beispiel 3

10 mg Polyacrylsäure (D) werden in 10 ml Acetatpuffer mit einem pH-Wert von 4,8 gelöst und mit 15 mg n-Cyclohexyl-N'-[2-morpholinyl-(4-ethyl)]-carbodiimid-m-p-toluolsulfonat versetzt. Man hält das Gemisch 1,5 Stunden bei 0°C. Die erhaltene Lösung wird mit 5 ml Puffer, der 5 mg 0-Streptolysin (A) und 5 mg Peroxidase (B) des Krens enthält, versetzt. Die Lösung wird 2 Stunden bei 20°C gehalten, wodurch man das Endprodukt erhält.

Beispiel 4

100 mg Agavosid G (A) werden in 5 ml Wasser vorgelegt und mit 100 mg Bromcyan (C) versetzt, wobei man durch Zugabe von 1 M NaOH-Lösung den pH-Wert bei 11 hält. Das Gemisch wird 30 Minuten lang bei 4°C gehalten, wonach man den pH-Wert durch Zugabe von Phosphorsäure auf 8,5 absenkt. 1 ml der so erhaltenen Lösung wird mit 3 ml einer 0,5 M Phosphatpufferlösung mit einem pH-Wert von 8,5 versetzt, die 6 mg alkalischer Phosphatase (B) enthält. Das Gemisch wird 12 Stunden lang bei 4°C gehalten, wodurch man eine Lösung des Endprodukts erhält.

Beispiel 5

500 mg Nistatin (A) und 250 mg Ethylen-Maleinsäureanhydrid-Copolymerisat (D) werden in 5 ml Dimethylformamid gelöst und 3 Stunden bei 50°C im Argonstrom gehalten. Das erhaltene Produkt wird mit 20 ml Ether ausgefällt und im Vakuum bei 20°C getrocknet. 1 ml einer Lösung von $\beta$-Galaktosidase (B) (2 mg/ml) in einem 0,2 M Phosphatpuffer mit einem pH-Wert von 7,5 wird mit 6 ml des erhaltenen Produkts versetzt und 12 Stunden bei 4°C gehalten. Man erhält eine Lösung des Endprodukts.

Beispiel 6

50 mg Aescin (A) und 100 mg N-Vinylpyrrolidon-Maleinsäureanhydrid-Copolymerisat (D) werden in 1 ml DMSO gelöst und 2 Stunden bei 100°C gehalten. Das erhaltene Produkt wird mit 3 ml Aceton ausgefällt und über $P_2O_5$ 4 Stunden lang bei 100°C im Vakuum getrocknet.

Danach wird 1 ml einer Lösung von 1 mg/ml von $\alpha$-Chymotrypsin (B) in 0,2 M Phosphatpuffer bei eine pH von 7,5 mit 8 mg des erhaltenen Produkts versetzt und 15 Stunden lang bei 6°C gehalten. Dadurch erhält man eine Lösung des Endprodukts.

Im vorliegenden Beispiel ist das erhaltene Produkt gekennzeichnet durch einen hohen Molargehalt an Nachweismittel A. Die Stufe der vorgängigen Herstellung des Nachweismittel A und höhermolekulares Vernetzungsmittel D enthaltenden Produkts ermöglicht eine maximale Erhaltung der enzymatischen Aktivität des Visualisierungsmittels B aufgrund der schonenden Bedingungen der Immobilisierung des Enzyms.

Verwendung der Verbindungen für den visuellen Cholesterinnachweis auf der Haut des Patienten:

Durch die mit Hilfe der erfindungsgemäß herstellbaren Verbindungen durchzuführende Diagnostik soll nicht die Cholesterinmenge genau gemessen werden, sondern es soll ermöglicht werden festzustellen, ob der Patient an Atherosklerose erkrankt ist, zur Risikogruppe gehört oder praktisch gesund ist. Um den Patienten einer dieser drei Gruppen zuordnen zu können, muß der für jede Gruppe kennzeichnende Cholesteringehalt in der Hornschicht der Epidermis der Haut festgestellt werden.

Zu diesem Zweck werden für jede erfindungsgemäß hergestellte Verbindung drei verschiedene Konzentrationen des affin-enzymatischen Mittels bereitet. Diese werden dann in Form von drei Punkten auf die Haut des Patienten, vorzugsweise auf die Handfläche aufgetragen. Die maximale Konzentration der jeweiligen Verbindung führt dann im weiteren bei sämtlichen Personen, darunter auch bei gesunden, die in der Haut den geringsten Cholesteringehalt aufweisen, zu einer Verfärbung des aufgebrachten Punktes bzw. Fleckens. Dieser wird dann gewissermaßen als Kontrollfleck für die Reaktion auf Cholesterin verwendet. Die Lösung mit der minimalen Konzentration der erfindungsgemäß hergestellten Verbindung führt nur bei Atherosklerosepatienten im klinischen Stadium, die einen maximalen Cholesteringehalt in der Haut aufweisen, zu einer Verfärbung. Die Lösung mit einer dazwischenliegenden Konzentration der erfindungsgemäß hergestellten Verbindung führt sowohl bei an Atherosklerose erkrankten Personen als auch bei solchen, die zur Risikogruppe zählen (erhöhter Cholesteringehalt) zu einer Verfärbung, nicht jedoch bei gesunden Personen.

Auf die Haut des Patienten werden somit drei unterschiedliche Konzentrationen der erfindungsgemäß erhaltenen Verbindung aufgetragen. Wird nur ein verfärbter Fleck festgestellt, ist die betreffende Person als gesund zu bezeichnen, bei zwei verfärbten Flecken ist der Patient der Risikogruppe zuzurechnen und bei drei Farbflecken der Gruppe der Atherosklerosepatienten im klinischen Stadium.

Für die Durchführung der Diagnostik reicht ein einige Quadratzentimeter großer Hautbezirk auf einem beliebigen Teil des Körpers aus. Besonders bevorzugt zu verwenden ist jedoch die Innenfläche der Hand, die leicht zugänglich ist und außerdem keine Talgdrüsen aufweist.

Die erfindungsgemäß hergestellte Verbindung wird in drei unterschiedlichen Konzentrationen auf die Hautoberfläche in einer Menge von 10 bis 20 µl aufgetragen und 1 Minute lang thermostatiert. Der Überschuß der Verbindung, der sich mit dem Cholesterin der Haut nicht verbindet, wird weggespült. Für die Sichtbarmachung der erfindungsgemäß hergestellten Verbindung, die sich mit dem Cholesterin der Haut verbunden hat, wird auf dieselben Hautbezirke das Substrat des entsprechenden Enzyms aufgetragen, das als Visualisierungsmittel B bei der Herstellung der Verbindung verwendet wurde, das seinerseits bei der Enzymreaktion mit der erfindungsgemäß hergestellten Verbindung das gefärbte Produkt bildet (s. T.T. Ngo und H.M. Lenhoff, "Enzyme-mediated Immunoassay" 1985, Plenum Press, New York, in der russischen Übersetzung dieses Buchs, S. 11, und A. Leninger "Biochemie", Verlag "Mir", Moskau, 1976, S. 198).

Je nach dem gewählten Substrat für die Enzymreaktion verfärbt sich die farblose Substratlösung rot, gelb, dunkelblau, grün, violett oder in anderer Weise oder die gefärbte Lösung entfärbt sich.

Wurde zum Beispiel als Visualisierungsmittel B Krenperoxidase verwendet, so kann als Substrat dieses Enzyms unmittelbar nach der Bereitung eine beliebige Lösung der nachfolgend aufgeführten Lösungen verwendet werden:

Lösung 1: ABTS (2,2′-Azinobis-(3-ethyl-benzthioazolin-6-sulfonsäure)) 1 mM, Wasserstoffperoxid 0,002 % in Phosphatcitratpuffer mit pH = 4,3;
Lösung 2: o-Phenylendiamin 4 mM, Wasserstoffperoxid 0,004 % in Phosphatcitratpuffer mit pH = 5;
Lösung 3: 3,3′,5,5′-Tetramethylenbenzidin 0,4 mM, Wasserstoffperoxid 0,004 % in Acetatpuffer mit pH = 6,0.

Es können aber auch andere Peroxidasesubstrate, wie sie in der Immunenzymanalyse in großem Umfange verwendet werden, zum Einsatz gelangen.

Wurde zum Beispiel als Visualisierungsmittel B bei der Herstellung der affin-enzymatischen Verbindung für den visuellen Cholesterinnachweis alkalische Phosphatase verwendet, kann als Substrat für dieses Enzym eine Lösung verwendet werden, die p-Nitrophenolphosphat (2,5 mM) und Magnesiumchlorid (0,5 mM) in einem Diethylaminpuffer (pH = 9,5) enthält, oder jedes andere Substrat einer alkalischen Phosphatase, das in der Immunenzymanalyse verwendet wird.

Die Ergebnisse der Untersuchung von 200 Personen mit verifizierter Diagnose, die in der Tabelle zusammengefaßt sind, ergaben eine hohe Korrelation zwischen dem diagnostischen Verfahren und der atherosklerotischen Schädigung der Gefäße.

Tabelle

| | Familiär bedingte Hyperlipidämie | Ischämie des Herzens | Hypertonie | Ischämie des Herzens + Hypertonie | Ischämie der unteren Extremitäten | Ischämie des Herzens + Hypertonie + Ischämie der unteren Extremitäten | Gesunde Personen |
|---|---|---|---|---|---|---|---|
| Zahl der Patienten | 9 | 7 | 44 | 30 | 8 | 66 | 30 |
| F | 5 | 2 | 13 | 12 | - | - | 15 |
| M | 4 | 5 | 31 | 18 | 8 | 66 | 15 |
| Durchschnittsalter | F-45 M-40 | F-55 M-47 | F-50 M-52 | F-54 M-52 | 48 | 54 | F-24 M-26 |
| Plasmacholesterin | $421,4\pm135,6$ | $252,0\pm54,6$ | $238,4\pm51,4$ | $290,5\pm78,6$ | $218,6\pm51,5$ | $239,6\pm41,3$ | $188,0\pm34,0$ |
| Atherogenitätsindex | $10,0\pm3,3$ | $5,4\pm1,9$ | $5,4\pm1,7$ | $7,6\pm2,9$ | $5,1\pm1,8$ | $5,7\pm2,6$ | $3,6\pm1,3$ |
| Erfindungsgemäße Hautprobe | 3 Flecken bei sämtlichen Personen | 3 Flecken bei 3 2 Flecken bei 4 | 3 Flecken bei 10 2 Flecken bei 3 | 3 Flecken bei 24 2 Flecken bei 6 | 3 Flecken bei 6 2 Flecken bei 2 | 3 Flecken bei sämtlichen Personen | 1 Fleck bei sämtlichen Personen |

F - Frauen,  M - Männer

EP 0 338 189 B1

**Patentansprüche**

1. Verfahren zur Herstellung von affin-enzymatischen Verbindungen zum visuellen Nachweis von Cholesterin auf der Hautoberfläche eines Patienten auf der Basis eines Nachweismittels A, das ein Affinans für Cholesterin darstellt, und eines Visualisierungsmittels B, dadurch **gekennzeichnet,** daß das Nachweismittel A, das ausgewählt ist aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder aus der Gruppe der Triterpenglykoside, die ein Aglykon der α-oder β-Amyryl-, Lupan-, Hopan-, Dammaran-, Linostan- oder Holostanreihe und ein Oligosaccharid aus 2 bis 8 verzweigten oder unverzweigten Resten enthalten, oder
aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen oder
aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder
aus der Gruppe der Polyenantibiotika Amphotericin B, Philipin oder Nistatin, oder
aus der Gruppe der Enzyme, die aus Cholesterinoxidasen, Cholesterinhydrogenasen und Cholesterinesterasen besteht,
durch chemische Methoden in wäßrigem Medium bei einem Molarverhältnis Nachweismittel A : Aktivator - 1:1 bis 1:10 und einer Konzentration des Nachweismittels A von 1 bis 20 mg/ml bei einer Temperatur von 0 bis 25°C und einem pH-Wert von 4 bis 11 1 bis 24 Stunden lang chemisch aktiviert wird und die erhaltene Lösung mit dem Visualisierungsmittel B, ausgewählt aus der Gruppe der Enzyme Acetylcholinesterase, Tyrosinase, Glukoso-6-phosphatdehydrogenase, Glukosooxidase, Glukosoamylase, Galaktosidase, Peroxidase, alkalische oder saure Phosphatase, α-Chymotrypsin oder Pyrophosphatase, im Molarverhältnis A:B - 20:1 bis 1:1 versetzt und die erhaltene Lösung 1 bis 24 Stunden lang bei einer Temperatur von 0 bis 25°C gehalten wird.

2. Verfahren zur Herstellung affin-enzymatischer Verbindungen zum visuellen Nachweis von Cholesterin auf der Hautoberfläche eines Patienten auf der Basis eines Nachweismittels A, das ein Affinans für Cholesterin darstellt und eines Visualisierungsmittels B, dadurch **gekennzeichnet,** daß das Visualisierungsmittel B, ausgewählt aus der Gruppe der Enzyme Acetylcholinesterase, Tyrosinase, Glukoso-6-phosphatdehydrogenase, Glukosooxidase, Glukosoamylase, Galaktosidase, Peroxidase, alkalische oder saure Phosphatase, α-Chymotrypsin oder Pyrophosphatase durch chemische Methoden in wäßrigem Medium bei einem Molarverhältnis Visualisierungsmittel B : Aktivator = 1:1 bis 1:10 und einer Konzentration des Visualisierungsmittels B von 1 bis 20 mg/ml bei einer Temperatur von 0 bis 25°C und einem pH-Wert von 4 bis 11 1 bis 24 Stunden lang chemisch aktiviert wird und die erhaltene Lösung entsprechend mit dem Nachweismittel A, ausgewählt
aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder aus der Gruppe der Triterpenglykoside, die ein Aglykon der α-oder β-Amyryl-, Lupan-, Hopan-, Dammaran-, Linostan- oder Holostanreihe und ein Oligosaccharid aus 2 bis 8 verzweigten oder unverzweigten Resten enthalten, oder
aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen, oder
aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder
aus der Gruppe der Polyenantibiotika Amphotericin B, Philipin oder Nistatin, oder
aus der Gruppe der Enzyme, die aus Cholesterinoxidasen, Cholesterinhydrogenasen und Cholesterinesterasen besteht, im Molarverhältnis A:B = 20:1 bis 1:1 versetzt wird und die Lösung bei O bis 25°C 1 bis 24 Stunden lang gehalten wird.

3. Verfahren zur Herstellung von affin-enzymatischen Verbindungen zum visuellen Nachweis von Cholesterin auf der Hautoberfläche eines Patienten auf der Basis eines Nachweismittels A, das ein Affinans für Cholesterin darstellt, und eines Visualisierungsmittels B, dadurch **gekennzeichnet,** daß das affine Nachweismittel A, das ausgewählt ist aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder aus der Gruppe der Triterpenglykoside, die ein Aglykon der α-oder β-Amyryl-, Lupan-, Hopan-, Dammaran-, Linostan- oder Holostanreihe und ein Oligosaccharid aus 2 bis 8 verzweigten oder unverzweigten Resten enthalten, oder

aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen, oder

aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder aus der Gruppe der Polyenantibiotika Amphotericin B, Philipin oder Nistatin, oder

aus der Gruppe der hochaffinen Enzyme, die aus Cholesterinoxidasen, Cholesterinhydrogenasen und Cholesterinesterasen besteht,

in einer wäßrigen Salz-Pufferlösung mit einem pH-Wert von 5 bis 9 bei einer Konzentration von A gleich 1 bis 20 mg/ml gelöst wird und die Lösung mit einem niedermolekularen asymmetrischen bifunktionellen Vernetzungsmittel C, ausgewählt aus der Gruppe Bromcyan, Trichlortriazin oder 2-Amino-4,6-dichlor-S-triazin im Molarverhältnis A: C = 1:0,5 bis 1:10 versetzt und die Lösung 1 bis 20 Stunden bei einer Temperatur von 0 bis 20°C gehalten wird, wonach das Visualisierungsmittel B, ausgewählt aus der Gruppe der Enzyme Acetylcholinesterase, Tyrosi-nase, Glukoso-6-phosphatdehydrogenase, Glukosooxidase, Gluko-soamylase, Galaktosidase, Peroxidase, alkalische oder saure Phosphatase, α-Chymotrypsin oder Pyrophosphatase, im Molarverhältnis A:B - 20:1 bis 1:1 zugesetzt und die Lösung 1 bis 48 Stunden bei einer Temperatur von 0 bis 20°C gehalten wird.

4. Verfahren zur Herstellung von affin-enzymatischen Verbindungen zum visuellen Nachweis von Cholesterin auf der Hautoberfläche eines Patienten auf der Basis eines Nachweismittels A, das ein Affinans für Cholesterin darstellt, und eines Visualisierungsmittels B, dadurch **gekennzeichnet,** daß man das Visualisierungsmittel B, ausgewählt aus der Gruppe der Enzyme Acetylcholinesterase, Tyrosinase, Glukoso-6-phopsphat-dehydrogenase, Glukosooxidase, Glukosoamylase, Galaktosidase, Peroxidase, alkalische oder saure Phosphatase, α-Chymotrypsin oder Pyrophosphatase in einer wäßrigen Salz-Pufferlösung mit einem pH von 5 bis 9 und bei einer Konzentration des Visualisierungsmittels B von 1 bis 20 mg/ml löst, das niedermolekulare asymmetrische bifunktionelle Vernetzungsmittel C, ausgewählt aus der Gruppe Bromcyan, Trichlortriazin oder 2-Amino-4,6-dichlor-S-triazin, im Molarverhältnis B:C = 1:0,5 bis 1:10, zusetzt und die Lösung 1 bis 20 Stunden bei einer Temperatur von 0 bis 20°C hält und sie dann entsprechend mit dem Nachweismittel A, das ausgewählt ist

aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder aus der Gruppe der Triterpenglykoside, die ein Aglykon der α- oder β-Amyryl-, Lupan-, Hopan-, Dammaran-, Linostan- oder Holostanreihe und ein Oligosaccharid aus 2 bis 8 verzweigten oder unverzweigten Resten enthalten, oder

aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen oder

aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder aus der Gruppe der Polyenantibiotika Amphotericin B, Philipin oder Nistatin, oder

aus der Gruppe der Enzyme, die aus Cholesterinoxidasen, Cholesterinhydrogenasen und Cholesterinesterasen besteht, im Molarverhältnis A:B = 20:1 bis 1:1 versetzt und die Lösung 1 bis 48 Stunden bei einer Tempeatur von 0 bis 20°C hält.

5. Verfahren zur Herstellung von affin-enzymatischen Verbindungen zum visuellen Nachweis von Cholesterin auf der Hautoberfläche eines Patienten auf der Basis eines Nachweismittels A, das ein Affinans für Cholesterin darstellt, und eines Visualisierungsmittels B, dadurch **gekennzeichnet,** daß das Nachweismittel A, das ausgewählt ist aus der Gruppe der Steroidglykoside, die als Aglykon das Cyclopentanperhydrophenantrenfragment der Furostanol- oder Spirostanolreihe sowie ein Oligosaccharidframgent mit 3 bis 10 unverzweigten oder verzweigten Monosacchariden enthalten, oder aus der Gruppe der Triterpenglykoside, die ein Aglykon der α- oder β-Amyryl-, Lupan-, Hopan-, Dammaran-, Linostan- oder Holostanreihe und ein Oligosaccharid aus 2 bis 8 verzweigten oder unverzweigten Resten enthalten, oder

aus der Gruppe der hydrophoben Proteine Proteine von Lysosomen und Mitochondrien, Fibrinogen, Immunoglobuline, Cerebron, Myoglobin, Trypsin, Cytochrom C, Cytochrom P-450 oder Apoeiweiße von Lipoproteinen, oder

aus der Gruppe der eiweißartigen Toxine Streptolysin o, Pneumolysin, Listeriolysin, θ-Toxin Cl., gewonnen aus Perfringens Typus A und C, δ-Toxin Cl. novyj Typus A und C, Hämolysin Cl. histolyticum, Hämolysin Cl. botulinum Typus C und D, Tetanolysin, Alveolysin, Türingeolysin oder Cytotoxin aus der Aktinie Metridium senile, oder aus der Gruppe der Polyenantibiotika Amphotericin B, Philipin oder Nistatin, oder

aus der Gruppe der hochaffinen Enzyme, die aus Cholesterinoxidasen, Cholesterinhydrogenasen und Cholesterinesterasen besteht, oder

ein Gemisch der genannten Stoffe mit dem Visualisierungsmittel B, ausgewählt aus der Gruppe der Enzyme Acetylcholinesterase, Tyrosinase, Glukoso-6-phosphatdehydrogenase, Glukosooxidase, Glukosoamylase, Galactosidase, Peroxidase, alkalische oder saure Phosphatase oder $\alpha$-Chymotrypsin im Molarverhältnis A:B = 20:1 bis 1:1 in einer wäßrigen Salzpufferlösung mit einem pH-Wert von 5 bis 9 bei einer Konzentration von A und B von jeweils 0,1 bis 20 mg/ml löst, die erhaltene Lösung mit dem höhermolekularen polyfunktionellen Vernetzungsmittel D, als welches Polysaccharide, Proteine oder synthetische Polymere, die eine beliebige funktionelle Gruppe, ausgewählt aus der Reihe primäres Amin, Carboxyl, Hydroxyl, Aldehyd, Halogenanhydrid, Mischanhydrid, Azid, Hydrazid, Maleimid, Isocyanat oder Epoxid, enthalten, verwendet werden, im Molarverhältnis (A+B):D = 1:0,5 bis 1:10 versetzt und die erhaltene Lösung 1 bis 20 Stunden bei einer Temperatur von 0 bis 20°C hält.

6. Verfahren nach Anspruch 5, dadurch **gekennzeichnet,** daß man als höhermolekulares Vernetzungsmittel D Copolymerisate der Acrylsäure oder des Maleinsäureanhydrids verwendet.

7. Verfahren nach Anspruch 6, dadurch **gekennzeichnet,** daß man als höhermolekulares Vernetzungsmittel D das N-Vinylpirrolidon-Maleinsäureanhydrid-Copolymerisat verwendet.

8. Verfahren nach einem der Ansprüche 1 bis 7, dadurch **gekennzeichnet,** daß man als Steroidglykoside, die als Nachweismittel A verwendet werden, Agavosid A, Agavosid B, Agavosid C, Agavosid D, Agavosid F, Agavosid G, Agavosid H, Trillin, Funcosid C, Funcosid D, Funcosid F, Funcosid G, Funcosid I, Dioscin, Gracillin, Protodioscin, Cicubasaponin, Juccosid E, Juccosid H, Lanatigonin, Desglukodigitonin, Digitonin, Gitonin, Rockosid C, Rockosid D, Rockosid E, Funcosid E, Alliumosid C, Polygonatosid, Tigogenintetraosid, Tigogeninhexaosid, Capsicosid, Desglukodesramnoparillin, Desglukoparillin, Parillin, Sarsaparillosid, Asparagosid C, Asparagosid D, Asparagosid G, Asparagosid H, Protoyuccosid H, Yuccosid B, Lanatigosid, Purpureagitosid, Gekogenin, Rockogenin, Diosgenin, Tigogenin, Protopolygonatosid, Tomatonin, Karataviosid A, Acantofilosid C, Alliofurosid A, Alliospirosid A, Theasaponin, Acantofilosid B, Tigonin (gesamt) oder Glykosid der Pflanze Calha polypetala verwendet.

9. Verfahren nach Anspruch 8, dadurch **gekenn zeichnet,** daß man als Nachweismittel A die Funcoside C, D, E, F, G oder I, Dioscin, Rockosid, Lanatigonin, Digitonin oder Tomatonin verwendet.

10. Verfahren nach Anspruch 9, dadurch **gekennzeichnet,** daß man als Nachweismittel A Digitonin oder Tomatonin verwendet.

11. Verfahren nach einem der Ansprüche 1 bis 7, dadurch **gekennzeichnet,** daß man als Triterpenglykoside für die Verwendung als Nachweismittel A Aescin, Avenacin, Theasaponin, $\alpha$-Hederin, Caulosid C, Cyclamin, Chinovin, Saponine der Zuckerrübe, Gypsosid oder Triterpenglykoside der Pflanze Fatsia japonica verwendet.

12. Verfahren nach einem der Ansprüche 1 bis 11, dadurch **gekennzeichnet,** daß man für die chemische Aktivierung des Nachweismittels A bzw. des Visualisierungsmittels B das an sich bekannte Carbodiimid-, Succinimid- oder Azidverfahren oder das Verfahren der Perjodatoxidierung verwendet.

13. Verfahren nach Anspruch 12, dadurch **gekennzeichnet,** daß man für die chemische Aktivierung des Nachweismittels A bzw. des Visualisierungsmittels B das Carbodiimidverfahren oder das Verfahren der Perjodatoxidierung verwendet.

14. Verfahren zur Herstellung von affin-enzymatischen Verbindungen zum visuellen Nachweis von Cholesterin auf der Hautoberfläche eines Patienten auf der Basis eines Nachweismittels A, das ein Affinans für Cholesterin darstellt, und eines Visualisierungsmittels B, dadurch **gekennzeichnet,** daß man das Nachweismittel A, das ausgewählt ist aus der Gruppe der Steroidglykoside, die als Aglykon das Cyclopentanperhydrophenantrenfragment der Furostanol- oder Spirostanolreihe sowie ein Oligosaccharidfragment mit 3 bis 10 unverzweigten oder verzweigten Monosacchariden enthalten, oder Triterpenglykoside, die ein Aglykon der $\alpha$-oder $\beta$-Amyryl-, Lupan-, Hopan-, Dammaran-, Linostan- oder Holostanreihe und ein Oligosaccharid aus 2 bis 8 verzweigten oder unverzweigten Resten enthalten, oder

Polyenantibiotika Amphotericin B, Philipin oder Nistatin, oder Gemische dieser Stoffe vorgängig in einem aprotischen polaren Lösungsmittel löst, wonach man das höhermolekulare polyfunktionelle Vernetzungsmittel D, ausgewählt aus der Gruppe Polysaccharide, Proteine oder synthetische Polymere, die eine beliebige funktionelle Gruppe, ausgewählt aus der Reihe primäres Amin, Carboxyl, Hydroxyl, Aldehyd, Hydrohalogenid, Mischanhydrid, Iminoether, Azid, Hydrazid, Maleimid, Isocyanat und Epoxid, bei einer Konzentration der Mittel A und D von jeweils 1 bis 20 mg/ml im Verhältnis A:D = 1:1 bis 1:10 zusetzt, die Lösung 0,5 bis 6 Stunden bei einer Temperatur von 20 bis 120°C hält, danach in an sich bekannter Weise das erhaltene Produkt (A-D) aus dem Lösungsmittel ausfällt, das erhaltene Produkt 4 bis 10 Stunden bei einer Temperatur von 80 bis 120°C im Vakuum über $P_2O_5$ hält, eine wäßrige Salz-Pufferlösung mit einem pH-Wert von 5 bis 9, in welcher das Visualisierungsmittel B, ausgewählt aus der Gruppe der Enzyme Acetylcholinesterase, Tyrosinase, Glukoso-6-phosphatdehydrogenase, Glukosooxidase, Glukosoamylase, Galaktosidase, Peroxidase, alkalische oder saure Phosphatase, $\alpha$-Chymotrypsin oder Pyrophosphatase, in einer Konzentration von 1 bis 10 mg/ml gelöst ist, mit dem erhaltenen trockenen Produkt (A-D) in einer Konzentration von 2 bis 20 mg/ml versetzt und die Lösung 2 bis 12 Stunden bei einer Temperatur von 4 bis 8°C hält.

15. Verfahren nach Anspruch 14, dadurch **gekennzeichnet,** daß man als aprotisches polares Lösungsmittel Dimethylsulfoxid, Dimethylformamid, Hexamethanol, ein Gemisch von Dimethylformamid und Toluol im Verhältnis 2:1 oder ein Gemisch aus Dimethylformamid und Hexanol im Verhältnis 2:1 verwendet.

16. Verfahren nach einem der Ansprüche 3 bis 5, 14 und 15, dadurch **gekennzeichnet,** daß man als wäßrige Salzlösungen Lösungen, ausgewählt aus der Borat-, Citrat- oder Phosphatreihe, verwendet, die eine Pufferwirkung im pH-Bereich von 5 bis 9 gewährleisten.

## Claims

1. Method of producing affine enzymatic compounds for the visual detection of cholesterol on the surface of the skin of a patient, on the basis of a detection agent A, having an affinity for cholesterol, and a visualisation agent B, characterised in that the detection agent A, which is selected from the group of steroid glycosides, which as an aglycon contain the cyclopentanperhydrophenantrene fragment of the furostanol or spirostanol series, as well as an oligosaccharide fragment with 3 to 10 unbranched or branched monosaccharides, or from the group of triterpene glycosides, Which contain an aglycon of the $\alpha$ or $\beta$ amyryl, lupan, hopan, dammaran, linostan or holostan series and an oligosaccharide of 2 to 8 branched or unbranched remnants, or from the group of hydrophobic proteins, proteins of lysosomes and mitochondria, fibrogenen, immunoglobins, cerebron, myoglobin, trypsin, cytochrome C, cytochrome P-450, or apo-proteins of lipoproteins, or from the group of protein-type toxins streptolysin o, pneumolysin, listeriolysin, $\theta$ toxin cl., obtained from perfringens type A and C, $\delta$-toxin cl. novyj type A and C, haemolysin cl. histolyticum, haemolysin cl. botulinum type C and D, tetanolysin, alveolysin, turingeolysin or cytotoxin from the actin metridium senile, or from the group of polyantibiotics amphotericin B, philipin or nystatin, or from the group of enzymes, comprising the cholesterol oxidases, cholesterol hydrogenases and cholesterol esterases, is activated by chemical means in an aqueous medium at a molecular ratio of detection agent A: activator = 1:1 to 1:10 and at a concentration of detection agent A of to 20 mg/ml at a temperature of 0 to 25°C and a pH value of 4 to 11, for 1 to 24 hours, and the obtained solution is mixed with visualisation agent B, selected from the group of enzymes acetyl cholinersterase, tyrosinase, glucoso-6-phosphate dehydrogenase, glucose oxidase, glucose amylase, galactosidase, peroxidase, alkaline or acid phosphatase, $\alpha$-chymotrypsin or pyrophosphatase, in a molecular ratio of A:B = 20:1 to 1:1, and the obtained solution kept at a temperature of 0 to 25°C for to 24 hours.

2. Method of producing affine enzymatic compounds for the visual detection of cholesterol on the surface of the skin of a patient, on the basis of a detection agent A, having an affinity for cholesterol, and a visualisation agent B, characterised in that the visualisation agent B, selected from the group of enzymes acetyl cholinesterase, tyronsinase, glucoso-6-hosphate dehydrogenase, glucose oxidase, glucose amylase, galactosidase, peroxidase, alkali or acid phosphatase, $\alpha$-chymotrypsin or pyrophosphatase is activated by chemical means in an aqueous medium with a molecular ratio of visualisation agent B:activator = 1:1 to 1:10 and a visualisation agent B concentration of 1 to 20 mg/ml, at a temperature of 0 to 25°C and a pH value of 4 to 11 for 1 to 24 hours, and the obtained solution is accordingly mixed with detection agent A, selected from the group of steroid glycosides, which as an aglycon contain the cyclopentanperhydrophenantrene fragment of the furostanol or spirostanol series, as well as an oligosaccharide fragment with 3 to 10 unbranched or branched monosaccharides, of from the group of triterpene glycosides, which contain an aglycon of the $\alpha$ or $\beta$ amyryl, lupan, hopan. dammaran, linostan or holostan series and an oligosaccharide of 2 to 8 branched or unbranched remnants, or from the group of hydrophobic proteins, proteins of lysosomes and mitochondria, fibrogenen, immunoglobins, cerebron, myoglobin, trypsin, cytochrome C, cytochrome P-450, or apo-proteins of lipoproteins, or from the group of protein-type toxins streptolysin o, pneumolysin, listeriolysin, $\theta$ toxin cl., obtained from perfringens type A and C, $\delta$-toxin cl. novyj type A and C, haemolysin cl. histolyticum, haemolysin cl. botulinum type C and D, tetanolysin, alveolysin. turingeolysin or cytotoxin from the actin metridium senile, or from the group of polyantibiotics amphotericin B, philipin or nystatin, or from the group of enzymes, comprising cholesterol oxidases, cholesterol hydrogenases and cholesterol esterases, at a molar ratio A:B = 20:1 to 1:1 and the solution is maintained at 0 to 25° for to 24 hours.

3. Method of producing affine enzymatic compounds for the visual detection of cholesterol on the surface of the skin of a patient, on the basis of a detection agent A, having an affinity for cholesterol, and a visualisation agent B, characterised in that the affine detection agent A, selected from the group of steroid glycosides, which as an aglycon contain the cyclopentanperhydrophenantren fragment of the furostanol or spirostanol series, as well as an oligosaccharide fragment with 3 to 10 unbranched or branched monosaccharides, or from the group of triterpene glycosides, which contain an aglycon of the α or β amyryl, lupan, hopan, dammaran, linostan or holostan series and an oligosaccharide of 2 to 8 branched or unbranched remnants, or from the group of hydrophobic proteins, proteins of lysosomes and mitochondria, fibrogenen, immunoglobins, cerebron, myoglobin, trypsin, cytochrome C, cytochrome P-450, or apo-proteins of lipoproteins, or from the group of protein-type toxins streptolysin o, pneumolysin, listeriolysin, θ toxin cl., obtained from perfringens type A and C, δ-toxin cl. novyj type A and C, haemolysin cl. histolyticum, haemolysin cl. botulinum type C and D, tetanolysin, alveolysin, turingeolysin or cytotoxin from the actin metridium senile, or from the group of polyantibiotics amphotericin B, philipin or nystatin, or from the group of high-affine enzymes, comprising cholesterol oxidases, cholesterol hydrogenases and cholesterol esterases, is dissolved in an aqueous saline buffer solution with a pH value of 5 to 9 and a concentration of A equal 1 to 20 mg/ml, and the solution is mixed with a low-molecular assymetric bi-functional cross-linking system C, selected from the group bromocyan, trichlorotriazin or 2-amino-4,6-dichloro-S-triazine in a molecular ratio of A:C = 1:0.5 to 1:10, and the solution is kept at a temperature of 0 to 20°C for 1 to 20 hours, whereafter the visualisation agent B, selected from the group of enzymes acetylcholinesterase, tyronsinase, glucoso-6-phosphate dehydrogenase, glucose oxidase, glucose amylase, galactosidase, peroxidase, alkali or acid phosphatase, α-chymotrypsin or pyrophosphatase, is added in a molecular ratio A:B = 20:1 to 1:1 and the solution is maintained at a temperature of 0 to 25°C for 1 to 48 hours.

4. Method of producing affine enzymatic compounds for the visual detection of cholesterol on the surface of the skin of a patient, on the basis of a detection agent A, having an affinity for cholesterol, and a visualisation agent B, characterised in that the detection agent B, selected from the group of enzymes acetyl cholinesterase, tyronsinase, glucoso-6-phosphate dehydrogenase, glucose oxidase, glucose amylase, galactosidase, peroxidase, alkali or acid phosphatase, α-chymotrypsin or pyrophosphatase is dissolved in an aqueous saline buffer solution with a pH of 5 to 9 and visualisation agent B concentration of 1 to 20 mg/ml, the low-molecular asymmetrical bi-functional cross-linking agent C, selected from the group bromocyan, trichlorotriazin or 2-amino-4,6-dichloro-S-triazine in a molecular ratio of B:C = 1:0.5 to 1:10 is added, and the solution is kept at a temperature of 0 to 20°C for 1 to 20 hours, and mixed with detection agent A, selected from the group of steroid glycosides, Which as an aglycon contain the cyclopentan-perhydrophenantrene fragment of the furostanol or spirostanol series, as well as an oligosaccharide fragment with 3 to 10 unbranched or branched monosaccharides, of from the group of triterpene glycosides, which contain an aglycon of the α or β amyryl, lupan, hopan, dammaran, linostan or holostan series and an oligosaccharide of 2 to 8 branched or unbranched remnants, or from the group of hydrophobic proteins, proteins of lysosomes and mitochondria, fibrogenen, immunoglobins, cerebron, myoglobin, trypsin, cytochrome C, cytochrome P-450, or apo-proteins of lipoproteins, or from the group of protein-type toxins streptolysin o, pneumolysin, listeriolysin, θ toxin cl., obtained from perfringens type A and C, δ-toxin cl. novyj type A and C, haemolysin cl. histolyticum, haemolysin cl. botulinum type C and D, tetanolysin, alveolysin turingeolysin or cytotoxin from the actin metridium senile, or from the group of polyantibiotics amphotericin B, philipin or nystatin, or from the group of enzymes, comprising cholesterol oxidases, cholesterol hydrogenases and cholesterol esterases, in a molecular ratio A:B = 20:1 to 1:1, and the solution is maintained at a temperature of 0 to 20°C for 1 to 48 hours.

5. Method of producing affine enzymatic compounds for the visual detection of cholesterol on the surface of the skin of a patient, on the basis of a detection agent A, having an affinity for cholesterol, and of a visualisation agent B, characterised in that the detection agent A, selected from the group of steroid glycosides, Which as an aglycon contain the cyclopentanperhydrophenantren fragment of the furostanol or spirostanol series, as well as an oligosaccharide fragment with 3 to 10 unbranched or branched monosaccharides, or from the group of triterpene glycosides, which contain an aglycon of the α or β amyryl, lupan, hopan, dammaran, linostan or holostan series and an oligosaccharide of 2 to 8 branched or unbranched remnants, or from the group of hydrophobic proteins, proteins of lysosomes and mitochondria, fibrogenen, immunoglobins, cerebron, myoglobin, trypsin, cytochrome C, cytochrome P-450, or apo-proteins of lipoproteins, or from the group of protein-type toxins streptolysin o, pneumolysin, listeriolysin, θ toxin cl., obtained from perfringens type A and C, δ-toxin cl. novyj type A and C, haemolysin cl. histolyticum, haemolysin cl. botulinum type C and D, tetanolysin, alveolysin, turingeolysin or cytotoxin from the actin metridium senile, or from the group of polyantibiotics amphotericin B, philipin or nystatin, or from the group of high-affine enzymes, comprising cholesterol oxidases, cholesterol hydrogenases and cholesterol esterases, or a mixture of said substances with visualisation agent B, selected from the group of enzymes acetyl cholinesterase, tyronsinase, glucoso-6-phosphate dehydrogenase, glucose oxidase, glucose amylase, galactosidase, peroxidase, alkali or acid phosphatase, $\alpha$-chymotrypsin or pyrophosphatase is dissolved in an aqueous saline buffer solution with a pH of 5 to 9 in a molecular ratio A:B = 20:1 to 1:1 with a concentration of A and B of 0.1 to 20 mg/ml, and the obtained solution is mixed with highly molecular, polyfunctional cross-linking agent D, for which polysaccharides, proteins or synthetic proteins of any functional group, selected from the series of primary amine, carboxyl, hydroxyl, aldehyde, halogen anhydride, mixed anhydride, azide, hydrazide, maleimide, isocyanate or epoxide, are used, in a molecular ratio (A=B):D = 1:0.5 to 1:10, the obtained solution being maintained at a temperature of 0 to 20°C for 1 to 20 hours.

6. Method according to claim 5, characterised in that copolymerisates of acrylic acid or maleic acid anhydride are used as the highly molecular cross-linking agent D.

7. Method according to claim 6, characterised in that the N-vinyl pirrolidon-maleic acid anhydride copolymerisate is used as the highly molecular cross-linking agent D.

8. Method according to any one of claims 1 to 7, characterised in that as steroid glycoside, used as detection agent A, agavoside A, agavoside B, agavoside C, agavoside D, agavoside F, agavoside G, agavoside H, trillin, funcoside C, funcoside D, funcoside F, funcoside G, funcoside I, dioscin, gracillin, protodioscin, cicubasaponin, juccoside E, juccoside H, lanatigonin, desglucodigitoni, digitonin, gitonin, rockoside C, rockoside D, rockoside E, funcoside E, alliumoside C, polygonatoside, tigogenintetraoside, tigogenin-hexoside, capsicoside, desglucodesramnoparillin, desglucoparillin, parillin, sarsaparilloside, asparagoside C, asparagoside D, asparagoside G, asporagoside H, protoyuccoside H, yuccoside B, lanatigoside, purple agitoside, gecogenin, rockogenin, diosgenin, tigogenin, protopoygonatoside, tomatonin, caratavoside A, acantofiloside C, alliofuroside A, alliospiroside A, theasaponin, acanto-philoside B, tigonin (total) or glycosides from the plant calha polypetala, are used.

9. Method according to claim 8, characterised in that as detection agent A, funcosides C, D, E, F, G or I, dioscin, rockoside, lanatigonin, digitonin or tomatonin are used.

10. Method according to claim 9, characterised in that as detection agent A, digitonin or tomatonin are used.

11. Method according to any one of claims 1 to 7, characterised in that as triterpene glycosides for use as detection agent A, aescin, avenacin, theasponin, $\alpha$-hederin, cauloside C, cyclamin, chinovin, sugar beet saponins, gypsoside or triterpene glycosides from the plant fatsia japonica are used.

12. Method according to any one of claims 1 to 11, characterised in that for the chemical activation of the detection agent A and the visualisation agent B, the known carbodiimide, succinimide or azide methods or the periodate oxidation method are used.

13. Method according to claim 12, characterised in that for the chemical activation of the detection agent A and the visualisation agent B, the carbodiimide method or the periodate oxidation method are used.

14. Method of producing affine enzymatic compounds for the visual detection of cholesterol on the surface of the skin of a patient, on the basis of a detection agent A, having an affinity for cholesterol, and visualisation agent B, characterised in that the detection agent A, Which is selected from the group of steroid glycosides. Which as an aglycon contain the cyclopentanperhydrophenantren fragment of the furostanol or spirostanol series, as well as an oligosaccharide fragment with 3 to 10 unbranched or branched monosaccharides, of from the group of triterpene glycosides, which contain an aglycon of the $\alpha$ or $\beta$ amyryl, lupan, hopan, dammaran, linostan or holostan series and an oligosaccharide of 2 to 8 branched or unbranched remnants, or polyantibiotics amphotericin B, philipin or nystatin, or mixtures of these substances, are first dissolved in an aprotic polar solvent, whereupon the highly molecular polyfunctional cross-linking agent D, selected from the group of polysaccharides, proteins or synthetic polymers, any functional group chosen from the series of primary amine, carboxyl, hydroxyl, aldehyde, hydrohalogenide, mixed anhydride, iminoether, azide, hydrazide, maleimide, isocyanate and epoxide, is added at a concentration of agents A and D of 1 to 20 mg/ml in a ratio A:D = 1:1 to 1:10, the solution then being kept at a temperature of 20 to 120°C for 0.5 to 6 hours, the obtained product (A-D) then being precipitated in a known manner from the solvent, the obtained product being kept in a vacuum over $P_2O_5$ at a temperature of 80 to 120°C for 4 to 10 hours, an aqueous saline buffer solution with a pH value of 5 to 9, in Which the visualisation agent B, selected from the group of enzymes acetyl cholinesterase, tyronsinase, glucoso-6-phosphate dehydrogenase, glucose oxidase, glucose amylase, galactosidase, peroxidase, alkali or acid phosphatase, α-chymotrypsin or pyrophosphatase is dissolved at a concentration of 1 to 10 mg/ml, is mixed the obtained dry product (A-D) at a concentration of 2 to 20 mg/ml, the solution being kept at a temperature of 4 to 8°C for 2 to 12 hours.

15. Method according to claim 14, characterised in that as the aprotic polar solvent, dimethylsulphoxide, dimethylformamide, hexamethanol, a mixture of dimethyl formamide and toluol at a ratio of 2:1 or a mixture of dimethyl formamide and hexanol at a ratio of 2:1 are used.

16. Method according to any one of claims 3 to 5, 14 and 15, characterised in that as the aqueous saline solution, solutions selected from the borate, citrate or phosphate series are used, which guarantee a buffer effect in the pH range 5 to 9.

**Revendications**

1. Procédé de fabrication de composés affines-enzymatiques pour la détection visuelle de cholestérol (cholestérine) sur la surface de la peau d'un patient à partir d'un agent de détection A, qui affiche une affinance pour le cholestérol, et d'un agent de visualisation B, ***caractérisé en ce que*** l'agent de détection A, qui est choisi parmi le groupe des glycosides stéroïdes qui contiennent comme aglycon le fragment cyclopentaneperhydrophénanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés,

ou, parmi le groupe des protéines hydrophobes, les protéines de lysosomes et de mitochondries, le fibrinogène, les immunoglobulines, la cérébrone, la myoglobine, la trypsine, le cytochrome C, le cytochrome P-450 ou les apoalbumines de lipoprotéines, ou parmi le groupe des toxines de type albumine, la streptolysine O, la pneumolysine, la listériolysine, les toxines θ Cl., fabriquées à partir de *Perfringens* types A et C, la toxine δ Cl. novyj types A et C, l'hémolysine Cl. *histolyticum,* l'hémolysine Cl. *botulinum* types C et D, la tétanolysine, l'alvéolysine, tŭringeolysine ou la cytotoxine de l'actinie *Metridium senile,*
ou, parmi le groupe des polyènes antibiotiques, l'amphotéricine B, la philipine ou la nistatine,
ou, parmi le groupe des enzymes qui est constitué des oxydases du cholestérol, des hydrogénases du cholestérol et des estérases du cholestérol,
est activé chimiquement par des méthodes chimiques, en milieu aqueux, avec un rapport molaire agent de détection A / activateur de 1/1 à 1/10, et une concentration de l'agent de détection A de 1 à 20 mg/ml, à une température de 0 à 25°C et un pH de 4 à 11, ceci pendant 1 à 24 heures, puis la solution obtenue est mélangée à l'agent de visualisation B, choisi parmi le groupe des enzymes acétylcholinestérase tyrosinase, glucoso-6-phosphatodéshydrogénase, glucosooxydase, glucosoamylase, galactosidase, péroxydase, phosphatase basique ou acide, α-chymotrypsine ou pyrophosphatase, selon un rapport molaire A/B de 20/1 à 1/1, et la solution obtenue est maintenue 1 à 24 heures à une température de 0 à 25°C.

2. Procédé de fabrication de composés affines-enzymatiques pour la détection visuelle de cholestérol à la surface de la peau d'un patient à partir d'un agent de détection A, qui affiche une affinance pour le cholestérol, et d'un agent de visualisation B, ***caractérisé en ce que*** l'agent de visualisation B, choisi parmi le groupe des enzymes acétylcholinestérase, tyrosinase, glucoso-6-phosphatodéshydrogénase, glucosooxydase, glucosoamylase, galactosidase, péroxydase, phosphatase basique ou acide, α-chymotrypsine ou pyrophosphatase, est activé chimiquement par des méthodes chimiques, en milieu aqueux, avec un rapport molaire agent de visualisation B/activateur de 1/1 à 1/10 et une concentration de l'agent de visualisation B de 1 à 20 mg/ml, à une température de 0 à 25°C et un pH de 4 à 11, ceci pendant 1 à 24 heures, puis la solution obtenue est mélangée à l'agent de détection A, qui est choisi parmi le groupe des glycosides stéroïdes qui contiennent comme aglycon le fragment cyclopentanepéthydrophenanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés,

ou, parmi le groupe des protéines hydrophobes, les protéines de lysosomes et de mitochondries, le fibrinogène, les immunoglobulines, la cérébrone, la myoglobine, la trypsine, le cytochrome C, le cytochrome P-450 ou les apoalbumines de lipoprotéines,
ou, parmi le groupe des toxines de type albumine, la streptolysine O, la pneumolysine, la listériolysine, les toxines θ Cl., fabriquées à partir de *Perfringens* types A et C, la toxine δ Cl. novyj types A et C, l'hémolysine Cl. *histolyticum,* l'hémolysine Cl. *botulinum* types C et D, la tétanolysine, l'alvéolysine, türingeolysine ou la cytotoxine de l'actinie *Metridium senile,*
ou, parmi le groupe des polyènes antibiotiques, l'amphotéricine B, la philipine ou la nistatine,
ou parmi le groupe des enzymes qui est constitué des oxydases du cholestérol, des hydrogénases du cholestérol et des estérases du cholestérol, selon un rapport molaire A/B de 20/1 à 1/1, et la solution est maintenue à une température de 0 à 25°C pendant 1 à 24 heures.

3. Procédé de fabrication de composés affines-enzymatiques pour la détection visuelle de cholestérol à la surface de la peau d'un patient à partir d'un agent de détection A, qui affiche une affinance pour le cholestérol, et d'un agent de visualisation B, ***caractérisé en ce que*** l'agent de détection A, qui est choisi parmi le groupe des glycosides stéroïdes qui contiennent comme aglycon le fragment cyclopentaneperhydrophénanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés,

ou, parmi le groupe des protéines hydrophobes, les protéines de lysosomes et de mitochondries, le fibrinogène, les immunoglobulines, la cérébrone, la myoglobine, la trypsine, le cytochrome C, le cytochrome P-450 ou les apoalbumines de lipoprotéines, ou, parmi le groupe des toxines de type albumine, la streptolysine O, la pneumolysine, la listériolysine, les toxines θ Cl., fabriquées à partir de *Perfringens* types A et C, la toxine δ Cl. novyj types A et C, l'hémolysine Cl. *histolyticum,* l'hémolysine Cl. *botulinum* types C et D, la tétanolysine, l'alvéolysine, türingeolysine ou la cytotoxine de l'actinie *Metridium senile,*
ou, parmi le groupe des polyènes antibiotiques, l'amphotéricine B, la philipine ou la nistatine,
ou parmi le groupe des enzymes hautement affines qui est constitué des oxydases du cholestérol, des hydrogénases du cholestérol et des estérases du cholestérol,
est dissous dans une solution saline tampon à un pH de 5 à 9 et une concentration de A égale à 1 à 20 mg/ml, et la solution est mélangée à un agent de réticulation bifonctionnel asymétrique C à faible poids moléculaire, choisi parmi le groupe du bromocyane, de la trichlorotriazine ou de la 2-amino-4,6-dichloro-S-triazine, selon un rapport molaire A/C de 1/0,5 à 1/10, et la solution est maintenue pendant 1 à 20 heures à une température de 0 à 20°C, après quoi l'agent de visualisation B, choisi parmi le groupe des enzymes acétylcholinestérase, tyrosinase, glucoso-6-phosphatodéshydrogénase, glucosooxydase, glucosoamylase, galactosidase, péroxydase, phosphatase basique ou acide, α-chymowypsine ou pyrophosphatase, est ajouté selon un rapport molaire A/B de 20/1 à 1/1, et la solution obtenue est maintenue de 1 à 48 heures à une température de 0 à 20°C.

4. Procédé de fabrication de composés affines-enzymatiques pour la détection visuelle de cholestérol à la surface de la peau d'un patient à partir d'un agent de détection A, qui affiche une affinance pour le cholestérol, et d'un agent de visualisation B, ***caractérisé en ce*** qu'on dissout l'agent de visualisation B, choisi parmi le groupe des enzymes acétylcholinestérase, tyrosinase, glucoso-6-phosphatodéshydrogénase, glucosooxydase, glucosoamylase, galactosidase, péroxydase, phosphatase basique ou acide, α-chymotrypsine ou pyrophosphatase, dans une solution saline tampon à un pH de 5 à 9 et avec une concentration de l'agent de visualisation B égale à 1 à 20 mg/ml, qu'on ajoute l'agent de réticulation bifonctionnel asymétrique C à faible poids moléculaire, choisi parmi le groupe du bromcyane, de la trichlorotriazine ou de la 2-amino-4,6-dichloro-S-triazine selon un rapport molaire B/C de 1/0,5 à 1/10, et qu'on maintient la solution pendant 1 à 20 heures à une température de 0 à 20°C, et qu'ensuite l'agent de détection A, qui est choisi parmi le groupe des glycosides stéroïdes qui contiennent comme aglycon le fragment cyclopentaneperhydrophénanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés, ou

parmi le groupe des protéines hydrophobes, les protéines de lysosomes et de mitochondries, le fibrinogène, les immunoglobulines, la cérébrone, la myoglobine, la trypsine, le cytochrome C, le cytochrome P-450 ou les apoalbumines de lipoprotéines,
ou, parmi le groupe des toxines de type albumine, la streptolysine O, la pneumolysine, la listériolysine, les toxines θ Cl., fabriquées à partir de *Perfringens* types A et C, la toxine δ Cl. novyj types A et C, l'hémolysine Cl. *histolyticum,* l'hémolysine Cl. *botulinum* types C et D, la tétanolysine, l'alvéolysine, türingeolysine ou la cytotoxine de l'actinie *Metridium senile,*
ou, parmi le groupe des polyènes antibiotiques, l'amphotéricine B, la philipine ou la nistatine,
ou parmi le groupe des enzymes qui est constitué des oxydases du cholestérol, des hydrogénases du cholestérol et des estérases du cholestérol, est ajouté selon un rapport molaire A/B de 20/1 à 1/1, et la solution obtenue est maintenue pendant 1 à 48 heures à une température de 0 à 20°C.

5. Procédé de fabrication de composés affines-enzymatiques pour la détection visuelle de cholestérol à la surface de la peau d'un patient à partir d'un agent de détection A, qui affiche une affinance pour le cholestérol, et d'un agent de visualisation B, *caractérisé en ce que* l'agent de détection A, qui est choisi parmi le groupe des glycosides stéroides qui contiennent comme aglycon le fragment cyclopentaneperhydrophénanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés,

ou, parmi le groupe des protéines hydrophobes, les protéines de lysosomes et de mitochondries, le fibrinogène, les immunoglobulines, la cérébrone, la myoglobine, la trypsine, le cytochrome C, le cytochrome P-450 ou les apoalbumines de lipoprotéines,
ou, parmi le groupe des toxines de type albumine, la streptolysine O, la pneumolysine, la listériolysine, les toxines θ Cl., fabriquées à partir de *Perfringens* types A et C, la toxine δ Cl. novyj types A et C, l'hémolysine Cl. *histolyticum,* l'hémolysine Cl. *botulinum* types C et D, la tétanolysine, l'alvéolysine, tŭringeolysine ou la cytotoxine de l'actinie *Metridium senile,*
ou, parmi le groupe des polyènes antibiotiques, l'amphotéricine B, la philipine ou la nistatine,
ou parmi le groupe des enzymes hautement affines qui est constitué des oxydases du cholestérol, des hydrogénases du cholestérol et des estérases du cholestérol,
ou un mélange desdites substances, est dissous avec l'agent de visualisation B, choisi parmi le groupe des enzymes acétylcholinestérase, tyrosinase, glucoso-6-phosphatodéshydrogénase, glucosooxydase, glucosoamylase, galactosidase, péroxydase, phosphatase basique ou acide ou α-chymotrypsine, selon un rapport molaire A/B de 20/1 à 1/1 dans une solution tampon saline à un pH de 5 à 9 et avec une concentration pour chacun de A et B de 0,1 à 20 mg/ml, la solution obtenue est mélangée à l'agent de réticulation polyfonctionnel à poids moléculaire élevé D, pour lequel on utilise des polysaccharides, des protéines ou des polymères de synthèse qui contiennent un groupe fonctionnel quelconque choisi dans la série amine primaire, carboxyle, hydroxyle, aldéhyde, anhydride d'halogène, anhydride mixte, azoture, hydrazide, maléimide, isocyanate ou époxyde, selon un rapport molaire (A+B)/D de 1/0,5 à 1/10, et la solution est maintenue pendant 1 à 20 heures à une température de 0 à 20°C.

6. Procédé selon la Revendication 5, *caractérisé en ce que,* comme agent de réticulation à poids moléculaire élevé D, on utilise des copolymères de l'acide acrylique ou de l'anhydride maléique.

7. Procédé selon la Revendication 6, *caractérisé en ce que,* comme agent de réticulation à poids moléculaire élevé D, on utilise le copolymère N-vinylpyrrolidone - anhydride maléique.

8. Procédé selon l'une des Revendications 1 à 7, *caractérisé en ce que,* comme glycosides stéroïdes qui sont employés comme agent de détection A, on utilise l'agavoside A, l'agavoside B, l'agavoside C, l'agavoside D, l'agavoside F, l'agavoside G, l'agavoside X, la trilline, le funcoside C, le funcoside D, le funcoside F, le funcoside G, le funcoside I, la dioscine, la gracilline, la protodioscine, la cicubasaponine, le juccoside E, le juccoside H, la lanatigonine, la desglucodigitonine, la digitonine, la gitonine, le rockoside C, le rockoside D, le rockoside E, le funcoside E, l'alliumoside C, le polygonatoside, le tigogéninetétraoside, le tigogéninhexaoside, le capsicoside, la desglucodesramnoparilline, la desglucoparilline, la parilline, le sarsaparilloside, l'asparagoside C, l'asparagoside D, l'asparagoside G, l'asparagoside H, le protoyuccoside H, le yuccoside B, le lanatigoside, le purpuréagitoside, la gécogénine, la rockogénine, la diosgénine, la tigogénine, le protopolygonatoside, la tomatonine, le caratavioside A, l'acantofiloside C, l'alliofuroside A, l'alliospiroside A, la théasaponine, l'acantotiloside B, la tigonine (tout) ou le glycoside de la plante *Calha polypetala.*

9. Procédé selon la Revendication 8, *caractérisé en ce que,* comme agent de détection A, on utilise les funcosides C, D, E, F, G ou I, la dioscine, le rockoside, la lanatigonine, la digitonine ou la tomatonine.

10. Procédé selon la Revendication 9, *caractérisé en ce que,* comme agent de détection A, on utilise la digitonine ou la tomatonine.

**11.** Procédé selon l'une quelconque des Revendications 1 à 7, *caractérisé en ce que,* comme glycoside triterpène pour l'utilisation en tant qu'agent de détection A, on utilise l'escine, l'avénacine, la théasaponine, l'α-hédérine, le cauloside C, la cyclamine, la quinovine (ou chinovine), la saponine de la betterave sucrière, le gypsoside ou des glycosides triterpènes de la plante *Fatsia japonica.*

**12.** Procédé selon l'une quelconque des Revendications 1 à 11, *caractérisé en ce que,* pour l'activation chimique de l'agent de détection A ou de l'agent de visualisation B, on utilise des procédés connus en soi utilisant le carbodiimide, le succinimide ou l'azoture, ou le procédé d'oxydation par les periodates.

**13.** Procédé selon l'une quelconque des Revendications 1 à 11, *caractérisé en ce que,* pour l'activation chimique de l'agent de détection A ou de l'agent de visualisation B, on utilise le procédé au carbodiimide ou le procédé d'oxydation par les periodates.

**14.** Procédé de fabrication de composés affines-enzymatiques pour la détection visuelle de cholestérol à la surface de la peau d'un patient à partir d'un agent de détection A, qui affiche une affinance pour le cholestérol, et d'un agent de visualisation B, *caractérisé en ce que* l'agent de détection A, qui est choisi parmi le groupe des glycosides stéroïdes qui contiennent comme aglycon le fragment cyclopentaneperhydrophénanthrène de la série du furostanol ou du spirostanol ainsi qu'un fragment oligosaccharide ayant de 3 à 10 monosaccharides ramifiés ou non-ramifiés, ou parmi le groupe des glycosides triterpènes qui contiennent un aglycon de la série α- ou β-amyryl, lupane, hopane, dammarane, linostane ou holostane et un oligosaccharide constitué de 2 à 8 résidus ramifiés ou non-ramifiés,

ou des polyènes antibiotiques, amphotéricine B, philipine ou nistatine, ou des mélanges de ces substances, est dissous en continu dans un solvant polaire aprotique, après quoi on ajoute l'agent de réticulation polyfonctionnel à poids moléculaire élevé D, choisi parmi le groupe des polysaccharides, des protéines ou des polymères de synthèse qui contiennent un groupe fonctionnel quelconque choisi dans la série amine primaire, carboxyle, hydroxyle, aldéhyde, hydrohalogénure, anhydride mixte, azoture, hydrazide, maléimide, isocyanate et époxyde, avec une concentration de chacun des agents A et D de 1 à 20 mg/ml selon un rapport A/D de 1/1 à 1/10, on maintient la solution 0,5 à 6 heures à une température de 20 à 120°C, après quoi le produit obtenu (A-D) précipite de manière connue depuis le solvant, on maintient le produit obtenu pendant 4 à 10 heures à une température de 80 à 120 °C sous vide sur $P_2O_5$, puis une solution tampon saline aqueuse d'un pH de 5 à 9, dans laquelle est dissous l'agent de visualisation B, choisi parmi le groupe des enzymes acétylcholinestérase, tyrosinase, glucoso-6-phosphatodéshydrogénase, glucosooxydase, glucosoamylase, galactosidase, péroxydase, phosphatase basique ou acide, α-chymotrypsine ou pyrophosphatase, avec une concentration de 1 à 10 mg/ml, est mélangée au produit sec (A-D) obtenu avec une concentration de 2 à 20 mg/ml, et on maintient la solution pendant 2 à 12 heures à une température de 4 à 8°C.

**15.** Procédé selon la Revendication 14, *caractérisé en ce que,* comme solvant polaire aprotique, on utilise du diméthylsulfoxyde, du diméthylformamide, de l'hexaméthanol, un mélange de diméthylformamide et de toluol selon un rapport de 2/1, ou un mélange de diméthylformamide et d'hexanol selon un rapport de 2/1.

**16.** Procédé selon l'une des Revendications 3 à 5, 14 et 15, *caractérisé en ce que,* comme solutions salines aqueuses, on utilise des solutions choisies parmi la série des borates, citrates ou phosphates, qui assurent un effet tampon dans le domaine de pH de 5 à 9.

EP 0 338 189 B1

Niedermolekulares
Vernetzungsmittel C

Cholesterin  Nachweis-  Visuali-  Höhermolekulares Vermittel A  sierungsmit-  netzungsmittel D
tel B

Fig. 1

Fig. 2

Fig. 3

Fig. 4

Hautoberfläche

21